# EUROPEAN PATENT SPECIFICATION

(11) **EP 3 359 195 B1**
(45) Date of publication and mention of the grant of the patent: **29.05.2024**
(21) Application number: 16777993.3
(22) Date of filing: 05.10.2016
(51) Int. Cl.: A61K 31/155, A61K 31/497, A61K 31/506, A61K 31/52, A61K 31/553, A61P 35/00, A61K 45/06

(54) **TREATMENT OF SEVERE SYSTEMIC MASTOCYTOSIS WITH MASITINIB**
BEHANDLUNG SCHWERER SYSTEMISCHER MASTOZYTOSE MIT MASITINIB
TRAITEMENT DE LA MASTOCYTOSE SYSTÉMIQUE SÉVÈRE PAR LE MASITINIB

(30) Priority: 05.10.2015 EP 15188430; 02.12.2015 EP 15197661
(43) Date of publication of application: 15.08.2018
(73) Proprietor: AB Science, 75008 Paris (FR)
(72) Inventor: MOUSSY, Alain, 75006 Paris (FR); KINET, Jean-Pierre, Lexington, Massachusetts 02420 (US)
(74) Representative: Icosa
(86) International application number: PCT/EP2016/073806
(87) International publication number: WO 2017/060308

(56) References cited:
- WO-A1-2012/059526
- CARLE PAUL ET AL: "Masitinib for the treatment of systemic and cutaneous mastocytosis with handicap: A phase 2a study", AMERICAN JOURNAL OF HEMATOLOGY, vol. 85, no. 12, 1 December 2010 (2010-12-01), pages 921-925, XP055013684, ISSN: 0361-8609, DOI: 10.1002/ajh.21894

## Description

### FIELD OF INVENTION

The present invention relates to the treatment of severe systemic mastocytosis in patients suffering from severe mast cell mediator release associated handicap.

### BACKGROUND OF INVENTION

### Mastocytosis

Mastocytosis is a term referring to a heterogeneous group of disorders characterized by abnormal mast cell accumulation in the skin and/or internal organs [Arock M, et al. Eur J Haematol. 2015 Jun; 94(6):474-90]. Systemic mastocytosis involves one or more extracutaneous organs (bone marrow, gastrointestinal tract, lymph nodes, and spleen), with or without skin involvement. Clinical signs and symptoms result from the release of chemical mediators and by infiltration of extracutaneous organs or skin by neoplastic mast cells. Mast cells are bone marrow derived cells that produce histamine and other substances causing allergic and anaphylactic reactions. Accumulation of mast cells in body organs can inhibit the functionality of the organ and eventually cause degeneration.

### Diagnosis and classification of mastocytosis

A summary of current conventions for defining patients with mastocytosis is provided in a recent review from Arock M, et al. and references therein [Arock M, et al. Eur J Haematol. 2015 Jun;94(6):474-90]. Patients with mastocytosis are commonly classified according to the proposal of the World Health Organization (WHO), distinguishing seven categories of the disease. An updated version of the WHO classification includes cutaneous mastocytosis (CM), indolent systemic mastocytosis (ISM), smoldering systemic mastocytosis (SSM), systemic mastocytosis with an associated clonal hematologic non-mast cell lineage disease (SM-AHNMD), aggressive systemic mastocytosis (ASM), mast cell leukemia (MCL), mast cell sarcoma (MCS), and extracutaneous mastocytoma. In most of these categories (CM, ISM, SM-AHNMD, ASM, MCL), subvariants have been identified, based on clinical and/or biological features [for example, see Table 1 of Arock, et al. Eur J Haematol. 2015 Jun;94(6):474-90]. WHO has also provided diagnostic criteria, with further categorization according to the presence of B-('Borderline Benign') findings and C-('Consider Cytoreduction') findings, which reflect the disease burden (B) and disease aggressiveness (C), respectively [for example, see Tables 2 and 3 of Arock, et al. Eur J Haematol. 2015 Jun;94(6):474-90]. For example, patients who have one or more C-findings are categorized as ASM or MCL and are candidates for therapeutic cytoreduction. Variants on the WHO diagnostic criteria are also used as appropriate.

### Mast cells

Mast cells are characterized by their heterogeneity, not only regarding tissue location and structure but also at functional and histochemical levels. Mast cell activation is followed by the controlled release of a variety of mediators that are essential for the defense of the organism against invading pathogens. By contrast, in the case of hyperactivation of mast cells, uncontrolled hypersecretion of these mediators is deleterious for the body. Mast cells produce a large variety of mediators categorized here into three groups:
- Preformed granule-associated mediators (histamines, proteoglycans, and neutral proteases);
- Lipid-derived mediators (prostaglandins, thromboxanes and leucotrienes);
- Various cytokines (including the interleukins: IL-1, IL-2, IL-3, IL-4, IL-5, IL-6, IL-8 and tumor necrosis factor alpha TNF-α, GM-CSF, MIP-1α, MIP-1β and IFN-γ).

Human mast cells constitutively express a number of receptors for different biological molecules. Among these receptors, whose ligation induces the activation of mast cells, the best known is the high affinity receptor for IgE (FcεRI). Binding of IgE-multivalent antigen complexes to FcεRI leads to receptor aggregation and internalization, signaling, and degranulation. This can be accompanied by the transcription of cytokine genes, thus, perpetuating the inflammatory response. Moreover, triggering of mast cells leads to the secretion of diverse pre-formed and/or de novo synthesized mediators, such as vasoactive amines (histamine, serotonin), sulfated proteoglycans, lipid mediators (prostaglandin D2, leucotrienes), growth factors, proteases, cytokines and chemokines as described previously. These mediators can, alone or in synergy with macrophage-derived and T cell-derived cytokines, generate a complex inflammatory response and induce the recruitment and activation of inflammatory cells to the site of degranulation.

### Role of c-Kit in mastocytosis

Stem cell factor (SCF), the ligand of the c-Kit receptor, is a major growth factor for mast cell survival, proliferation, differentiation, adhesion and degranulation processes [Reber et al., Eur J Pharmacol 2008;533:327-340], with SCF-dependent activation of c-Kit critical for mast cell homeostasis and function. Binding of SCF to the c-Kit receptor induces c-Kit dimerization followed by its transphosphorylation, leading to the recruitment and activation of various intracytoplasmic substrates. These activated substrates induce multiple intracellular signaling pathways responsible for cell proliferation and activation. The symptoms of mastocytosis are caused by the uncontrolled accumulation of mast cells and release of their mediators. Deregulated activity of the SCF/c-Kit pathway in mastocytosis is related to mutations in the c-Kit receptor. It has been shown that between 70% and 90% of patients with systemic forms of mastocytosis carry the gain-of-function Asp-816-Val (D816V) mutation in the kinase (phosphotransferase) domain of c-Kit, with the remainder (10% and 30%) carrying mutations in other domains of the molecule, such as the transmembrane domain. This mutation is associated with ligand-independent constitutive activation of c-Kit signaling, leading to uncontrolled mast cell proliferation, resistance to apoptosis and mediator release.

### Treatment of mastocytosis

The treatment of mastocytosis, and in particular the long-term management of mastocytosis, remains a challenge to clinicians because of the diversity and complexity of the disease itself and the lack of a standard and highly effective therapy. None of the approved drugs represent a cure for the disease and no therapy available effectively destroys the mast cells responsible for mastocytosis; moreover, their efficacy is limited and may decrease over time, with undesirable side effects reported. In general, management of patients within all categories of mastocytosis includes: (i) avoidance of factors triggering acute mediator release, (ii) symptomatic treatment of acute mast cell mediator release, (iii) treatment of chronic mast cell mediator release, and if indicated (iv) an attempt to treat organ infiltration by mast cells. However, even with the help of currently available symptomatic treatments, mastocytosis can have a profoundly negative impact on quality of life.

In a retrospective study, Mayo Clinic patients who met the 2008 WHO diagnostic criteria for mastocytosis and had received at least one of four cytoreductive drugs including: interferon-alpha with or without prednisone (IFN-α), hydroxyurea, imatinib mesilate or Cladribine (2-CdA), were evaluated for response [Lim et al., Am J Hemato. 2009; 84:790-4]. Response to each therapy was assessed via published consensus criteria; briefly, complete response (CR) to treatment comprised complete resolution of all clinical symptoms and signs lasting for at least 1 month; major response (MR) comprised a more than 50% improvement in symptoms and signs; partial response (PR) comprised a 10% to 50% improvement. Overall response rate was defined as the sum of CR, MR and PR. The corresponding overall response rates for those patients with ISM (N=22) were 60%, 0%, 14%, and 56%, respectively. Considering the entire evaluable study population (N=80), which included patients with more aggressive forms of mastocytosis such as ASM, SM-AHNMD, and MCL, the corresponding overall response rate (with major response shown in brackets) were 53% (18%), 19% (0%), 18% (9%), and 55% (37%), respectively.

Overall, the major response rate for these four cytoreductive agents was considered suboptimal for first-line therapy in mastocytosis. It was also noted that the degree and duration of response from these and other drugs remained inadequate, with relatively short-lived response, development of resistance and reoccurrence of symptoms, as well as restricted usefulness due to detrimental side-effects. Novel drugs are required to address this unmet need.

A recent publication reviewed major treatment options that are currently available to control mediator release and effects (antimediator therapies) or the expansion of neoplastic mast cells in advanced systemic mastocytosis (cytoreductive and targeted treatment), including: antihistamines, antileukotrienes, glucocorticoids, `mast cell-stabilizing agents', epinephrine, omalizumab, allogeneic stem cell transplantation, cladribine (2-CdA), interferon-alpha (IFN-α), and small-molecule targeted therapies such as imatinib, dasatinib, bosutinib, ponatinib, semaxanib, and midostaurin [Arock M, et al. Eur J Haematol. 2015 Jun;94(6):474-90]. It was reported that none of the available drugs could clearly be established as a preferred first-line therapy in systemic mastocytosis due to a general lack of evidence from randomized controlled studies or because drugs were of limited usefulness due to detrimental side-effects and/or insufficient response.

Hence, there exists a continuing need to identify new targeted drugs that possess greater inhibitory action against c-Kit, with improved selectivity to minimize side effects, capable of inhibiting mast cell survival and release of mast cell mediators for treatment of mastocytosis with mast cell mediator release associated handicap, and in particular systemic mastocytosis.

### Mast cell mediator release symptoms

Patients suffering from mastocytosis often experience symptoms resulting from the constitutive activation of mast cells and release of their mediators. Collectively, these are referred to as "mast cell mediator release symptoms". Symptoms may include: asthenia (fatigue), pruritus, flushing, micturition frequency (pollakiuria), stool frequency (diarrhea), and psychological impact of the disease, particularly depression [Hermine O, et al., PLoS ONE. 2008;3:e2266]. Each clinical symptom can be objectively measured by frequency or via an appropriate rating scale, although these do not form any part of formal diagnosis of systemic mastocytosis. However, no formally established thresholds for categorizing the burden and severity of disability in systemic mastocytosis related to mast cell mediator release associated handicaps exist. This is in part because the perception of handicap is highly dependent on the patient's lifestyle and environment; that is to say, identical symptoms may be perceived as a handicap resulting in significant detriment to quality-of-life for one patient, yet impact on another patient merely as a minor annoyance. Thus, beyond the established WHO methods for diagnosing mastocytosis, treatment decisions for mastocytosis relies upon the patient's and physician's assessment of symptoms and therefore assessment of mast cell mediator release associated handicap.

To make the patient's and physician's assessment of handicap severity objective and measurable, it is usually relied on the following measuring methods/rating scales:
- For flushes: number of flushes per week;
- For diarrhea: number of stools per day;
- For pollakiuria: number of micturitions per day;
- For depression: the score on the Hamilton rating scale [Hamilton M. J Neurol Neurosurg Psychiatry. 1960;23:56-62 ; Hedlund JL, et al. J Oper Psychiatry. 1979;10:149-161]. The Hamilton rating scale (HAMD-17, also referred to as HAM-D, HAM-D17, HRSD) remains a reference measure to evaluate depression in research concerning somatic patients. HAMD-17 is composed of 17 items scored 0-4 (depressed mood, guilt, suicide, psychic and somatic anxiety, psychomotor retardation, agitation, hypochondriasis, work and interests impairment) or 0-2 (early, middle and late insomnia, gastrointestinal, somatic general, genital, loss of weight and loss of insight items) according to the absence, presence and seriousness of the symptom. HAMD-17 categories are defined according to the scoring manual as: 0-7 Normal, 8-13 Mild Depression, 14-18 Moderate Depression, 19-22 Severe Depression, ≥ 23 Very Severe Depression [Hamilton, M: A rating scale for depression, Journal of Neurology, Neurosurgery, and Psychiatry 23:56-62, 1960]. Hence a HAMD-17 score ≥ 19 corresponds to a severe mast cell mediator release associated handicap;
- For fatigue: the score on the Fatigue Impact scale (FIS) (The Fatigue Impact Scale was designed as a fatigue-specific measure for patients in primary care setting and also as a research tool [Fisk JD, et al. Clin Infect Dis. 1994;18:S79-83]. It can be used as a clinical measure to guide intervention or treatment, and to assess change over time. FIS consists of 40 questions within these three groups: cognitive, physical, and psychosocial functioning. The person who is taking the test rates the extent to which fatigue causes problems in his/her life. The Fatigue Impact Scale (FIS) is one of the most widely used tools, although there now exist modified versions [the modified Fatigue Impact Scale (MFIS), the daily FIS, the unidimensional FIS and the abbreviated MFIS]. Other scores for measuring fatigue include Fatigue Severity Scale (FSS), Fatigue Symptom Inventory (FSI), Brief Fatigue Inventory (BFI) or Multidimensional Assessment of Fatigue (MAF), as described by Whitehead [Journal of Pain and Symptom Management, 2009, 37(1): 107-128];
- For pruritus: the score on a numerically amended version of the scale taught in Hermine O, et al., PLoS ONE. 2008;3:e2266. (The presence of pruritus and its score can be assessed in compliance with Hermine O, et al., PLoS ONE. 2008;3:e2266).

Depending on the score and thus on the severity of the handicap, mastocytosis may be classified as mild, moderate or severe (or as systemic mastocytosis associated with mild, moderate or severe handicap), with the latter category representing a disabling even life-threatening problem.

The treatment of systemic mastocytosis, and more specifically of smoldering systemic mastocytosis or indolent systemic mastocytosis with severe mast cell mediator release associated handicap, remains a challenge to clinicians because of the diversity and complexity of the disease itself and the lack of standard and clinically meaningful effective therapy.

None of the known approved drugs appear to represent a cure for systemic mastocytosis and no available therapy appears to regulate the activation of mast cells responsible for severe systemic mastocytosis. Moreover, the efficacy of known drugs is limited and may decrease over time, with undesirable side effects reported. It therefore results that there is no indication, in the prior art, that any of the known drugs could actually provide a reliable treatment for alleviating the symptoms of patients suffering from severe systemic mastocytosis, more particularly for treating smoldering systemic mastocytosis or indolent systemic mastocytosis in patients suffering from severe mast cell mediator release associated handicap. Thus, there exists a continuing need to identify new targeted drugs that possess greater inhibitory action against the uncontrolled activation of mast cells which result in the occurrence of severe systemic mastocytosis, while being also capable of minimizing side effects.

The use of masitinib, a small molecular weight tyrosine kinase inhibitor capable of inhibiting tyrosine kinase activity of c-Kit, Lyn, and Fyn or of a pharmaceutical salt or solvate thereof has shown promising preliminary results in the treatment of systemic or cutaneous mastocytosis with related symptoms in human patients with an overall patient assessment (OPA)≥1 (see for example, the patent application US 13/881,043 and also WO 2012/059526 or Paul C, et al., Am J Hematol. 2010;85(12):921-925). Both WO 2012/059526 and Paul C, et al. assessed the effect of masitinib on mastocytosis associated handicaps considered independently and concluded that masitinib was able to reduce mast cell mediator release associated handicaps in patients suffering from indolent mastocytosis.

The present invention thus relates to masitinib or a pharmaceutically acceptable salt or solvate thereof for use in the treatment of severe systemic mastocytosis in a human patient, wherein said severe systemic mastocytosis is associated with at least two mast cell mediator release associated handicaps selected from a pruritus score ≥ 9; a number of flushes per week ≥ 8; a Hamilton rating scale for depression (HAMD-17) score ≥ 19; a number of stools per day ≥ 4; a number of micturition per day ≥ 8; and a fatigue (asthenia) score measured by Fatigue Impact Scale (FIS) ≥ 75, and wherein at least two mast cell mediator release associated handicaps are selected from a pruritus score ≥ 9; a number of flushes per week ≥ 8; and a HAMD-17 score ≥ 19, wherein said systemic mastocytosis is smoldering systemic mastocytosis or indolent systemic mastocytosis.

In relation to the present invention, the term "treatment" (and its various grammatical forms) refers to preventing, curing, reversing, attenuating, alleviating, minimizing, suppressing or halting the deleterious effects of a disease state, disease progression, disease causative agent (e.g., bacteria or viruses) or other abnormal condition. For example, treatment may involve alleviating a symptom (i.e., not necessary all symptoms) of a disease or attenuating the progression of a disease.

The inventors have surprisingly shown that masitinib mesilate provides therapeutic benefit to a highly distinct subpopulation of systemic mastocytosis patients, with severe mast cell mediator release associated handicaps serving as an independent predictor factor for treatment efficacy, in particular the severe handicaps of pruritus and flushes (see Examples 2 and 3).

### SUMMARY

The present invention relates to masitinib or a pharmaceutically acceptable salt or solvate thereof for use in the treatment of severe systemic mastocytosis in a human patient, wherein said severe systemic mastocytosis is associated with at least two mast cell mediator release associated handicaps selected from a pruritus score ≥ 9; a number of flushes per week ≥ 8; a Hamilton rating scale for depression (HAMD-17) score ≥ 19; a number of stools per day ≥ 4; a number of micturition per day ≥ 8; and a fatigue (asthenia) score measured by Fatigue Impact Scale (FIS) ≥ 75, and wherein at least two mast cell mediator release associated handicaps are selected from a pruritus score ≥ 9; a number of flushes per week ≥ 8; and a HAMD-17 score ≥ 19, wherein said systemic mastocytosis is smoldering systemic mastocytosis or indolent systemic mastocytosis.

In some embodiments, said severe systemic mastocytosis is associated with a pruritus score ≥ 9 and a number of flushes per week ≥ 8.

In some embodiments, said severe systemic mastocytosis is associated with the three following mast cell mediator release associated handicaps: a pruritus score ≥ 9; a number of flushes per week ≥ 8; and a HAMD-17 score ≥ 19.

In some embodiments, said severe systemic mastocytosis is further associated with the mast cell mediator release associated handicap of a fatigue (asthenia) score measured by FIS ≥ 75 or any equivalent cut-off determined using the Fatigue Severity Scale (FSS), Fatigue Symptom Inventory (FSI), Brief Fatigue Inventory (BFI) or Multidimensional Assessment of Fatigue (MAF).

In some embodiments, said smoldering systemic mastocytosis or indolent systemic mastocytosis is defined by the World Health Organization (WHO) diagnostic criteria.

In some embodiments, said smoldering systemic mastocytosis or indolent systemic mastocytosis relates to a disease for which any of the following four criteria are fulfilled at baseline or before baseline:
- presence of mast cells in any bone marrow biopsy or aspirate associated with at least one sign of abnormality, wherein said abnormal signs are selected from: more than 1% infiltration of mast cells in bone marrow; aggregates of more than 15 mast cells in bone marrow that can be described by the following words: nodules, seats, clusters, foci, or granuloma; more than 25% atypical mast cells in a sample of bone marrow; abnormal mast cells in the sample of bone marrow with microscopic testing that can be described by the following words: spindled, abnormal, atypical, fusiform, dystrophic, pathologic, dysmorphic; abnormal immunohistochemistry signs: mast cells in bone marrow express CD2 and/or CD25 present; and c-Kit point mutation at codon 816 (c-Kit 816) in bone marrow; or
- detection of c-Kit 816 in bone marrow without evidence of mast cells in bone marrow and detection of c-Kit 816 in skin biopsy justifying clonality; or
- presence of mast cells in bone marrow biopsy or aspirate without signs of mast cell abnormality; or
- excess of mast cells detected in digestive organs in addition to excess of mast cells in the skin.

In some embodiments, said human patient has a positive D816V c-Kit mutation status.

In some embodiments, said pharmaceutically acceptable salt or solvate of masitinib is masitinib mesilate.

In some embodiments, said masitinib or a pharmaceutically acceptable salt or solvate thereof, preferably masitinib mesilate, is for administration at a dose of 1.5, 3.0, 4.5, 6.0, 7.5, or 9.0 mg/kg/day (mg per kilo body weight per day); preferably at an initial dose of 3.0 mg/kg/day during at least 4 weeks, then 4.5 mg/kg/day during at least 4 weeks, and at 6 mg/kg/day thereafter, with each dose escalation being subjected to toxicity controls.

In some embodiments, said masitinib or a pharmaceutically acceptable salt or solvate thereof, preferably masitinib mesilate, is for administration in two daily intakes.

In some embodiments, said masitinib or a pharmaceutically acceptable salt or solvate thereof, preferably masitinib mesilate, is for oral administration.

In some embodiments, said masitinib or a pharmaceutically acceptable salt or solvate thereof, preferably masitinib mesilate, is comprised in a pharmaceutical composition in an amount of at least 50 mg and less than 600 mg, preferably of at least 100 mg and less than 400 mg.

The invention aims to solve the technical problem of providing an active ingredient for the treatment of severe systemic mastocytosis.

The invention aims to provide an efficient treatment for such a disease at an appropriate dose, route of administration and daily intake.

The invention also aims to solve the technical problem of providing an active ingredient that improves prior art methods for the treatment of severe systemic mastocytosis.

According to an embodiment, said systemic form of mastocytosis is defined by the World Health Organization (WHO) classification and diagnostic criteria [Arock M, et al. Eur J Haematol. 2015 Jun;94(6):474-90], or said systemic form of mastocytosis is defined using two modified diagnostic criteria, referred to hereafter as the 'AB Science Systemic Mastocytosis (ABSSM) criteria', or as the `Protocol AB06006 v7 criteria', as defined herein below.

In a first embodiment, systemic mastocytosis is defined according to the World Health Organization (WHO) classification and diagnostic criteria. A diagnosis of systemic mastocytosis is made according to the presence of B-('Borderline Benign') findings and C-('Consider Cytoreduction') findings, which reflect the disease burden (B) and disease aggressiveness (C), respectively [for example, see Tables 2 and 3 of Arock, et al. Eur J Haematol. 2015 Jun;94(6):474-90]. Patients who have systemic mastocytosis without B- or C-findings and without signs of an AHNMD are diagnosed as indolent systemic mastocytosis (ISM). Patients who have two or more B-findings, but no C-findings, are diagnosed with smoldering systemic mastocytosis (SSM), a separate systemic mastocytosis category initially considered a subtype of ISM defined by a huge mass of neoplastic mast cells.

In a second embodiment, the term systemic mastocytosis is defined by the ABSSM diagnostic criteria, and thus relates to a disease for which any the following four criteria are fulfilled at baseline or before baseline:
- Presence of mast cells in any bone marrow biopsy or aspirate associated with at least one sign of abnormality, wherein said abnormal signs are:
   - More than 1% infiltration of mast cells in bone marrow;
   - Aggregates of more than 15 mast cells in bone marrow that can be described by the following words: nodules, seats, clusters, foci, or granuloma;
   - More than 25% atypical mast cells in a sample of bone marrow;
   - Abnormal mast cells in the sample of bone marrow with microscopic testing that can be described by the following words: spindled, abnormal, atypical, fusiform, dystrophic, pathologic, dysmorphic;
   - Abnormal immunohistochemistry signs: mast cells in bone marrow express CD2 and/or CD25 present;
   - c-Kit point mutation at codon 816 (c-Kit 816) in bone marrow.
- Detection of c-Kit 816 in bone marrow without evidence of mast cells in bone marrow and detection of c-Kit 816 in skin biopsy justifying clonality;
- Presence of mast cells in bone marrow biopsy or aspirate without signs of mast cell abnormality;
- Excess of mast cells detected in digestive organs in addition to excess of mast cells in the skin.

In a third embodiment, the term systemic mastocytosis is defined by the Protocol AB06006 version 7.0 (v7) diagnostic criteria and thus relates to a disease for which the following criteria are fulfilled:
- Patient with one of the following documented mastocytosis as per WHO classification:
   - Smoldering Systemic Mastocytosis;
   - Indolent Systemic Mastocytosis; and/or
- Patient with documented mastocytosis and evaluable disease based upon histological criteria including typical infiltrates of mast cells in a multifocal or diffuse pattern in skin and/or bone marrow biopsy.

In one embodiment said patient with systemic mastocytosis, as defined by the ABSSM, has a positive D816V c-Kit mutation status.

In another embodiment said patient with systemic mastocytosis, as defined by the ABSSM, has a negative D816V c-Kit mutation status.

According to the present invention, said severe systemic mastocytosis is associated with at least two mast cell mediator release associated handicaps selected from the list comprising pruritus score ≥ 9, number of flushes per week ≥ 8, and depression measured by the Hamilton rating scale ≥ 19.

In one embodiment, said severe systemic mastocytosis is associated with pruritus score ≥ 9 and number of flushes per week ≥ 8.

In one embodiment, said severe systemic mastocytosis is associated with the three following mast cell mediator release associated handicaps: pruritus score ≥ 9, number of flushes per week ≥ 8, and depression measured by the Hamilton rating scale ≥ 19.

In one embodiment, said severe systemic mastocytosis is further associated with the mast cell mediator release associated handicap of asthenia (fatigue) with a fatigue score measured by Fatigue Impact Scale (FIS) ≥ 75 (or any equivalent cut-off determined using the FSS, FSI, BFI or MAF).

In one embodiment, said severe systemic mastocytosis is associated with the three following mast cell mediator release associated handicaps: pruritus score ≥ 9, number of flushes per week ≥ 8, and depression measured by the Hamilton rating scale ≥ 19 and is further associated with the mast cell mediator release associated handicap of asthenia (fatigue) with a fatigue score measured by Fatigue Impact Scale (FIS) ≥ 75 (or any equivalent cut-off determined using the FSS, FSI, BFI or MAF).

In one embodiment, said masitinib or a pharmaceutically acceptable salt or solvate thereof, preferably masitinib mesilate is to be administered orally.

In one embodiment, said masitinib or a pharmaceutically acceptable salt or solvate thereof, preferably masitinib mesilate is to be administered twice a day (i.e. in two daily intakes).

In one embodiment, said masitinib or a pharmaceutically acceptable salt or solvate thereof, preferably masitinib mesilate, is for administration at a daily dose of between 1.5 to 9.0 mg/kg/day; for example, 1.5, 3.0, 4.5, 6.0, 7.5, or 9.0 mg/kg, more preferably 3.0, 4.5 or 6 mg/kg/day (mg per kg bodyweight per day).

In one embodiment, said masitinib or a pharmaceutically acceptable salt or solvate thereof is to be dose escalated by increments of 1.5 mg/kg/day to reach a maximum of 9.0 mg/kg/day, more preferably 6 mg/kg/day. Each dose escalation is to be subjected to toxicity controls with an absence of any toxicity events permitting dose escalation to occur.

In one embodiment, dose escalation of masitinib or a pharmaceutically acceptable salt or solvate thereof is to occur at any time-point after at least 4 weeks after the initial dose has been administered and prior to 26 weeks after the initial dose has been administered; for example at week-4, week-8, week-12, week-16, week-20, or week-24.

As an example, masitinib or a pharmaceutically acceptable salt or solvate thereof is to be initially administered per os, preferably in two daily intakes, at a dose of 3 mg/kg/day during 4 weeks, then 4.5 mg/kg/day during 4 weeks, and then 6 mg/kg/day thereafter. In another example, masitinib or a pharmaceutically acceptable salt or solvate thereof is to be initially administered per os, preferably in two daily intakes, at a dose of 4.5 mg/kg/day during 12 weeks, and then 6 mg/kg/day thereafter.

In one embodiment, masitinib or a pharmaceutically acceptable salt or solvate thereof, preferably masitinib mesilate, is to be initially administered at a dose of 3.0 mg/kg/day during at least 4 weeks, then 4.5 mg/kg/day during at least 4 weeks, and at 6 mg/kg/day thereafter, with each dose escalation being subjected to toxicity controls.

In one embodiment, masitinib or a pharmaceutically acceptable salt or solvate thereof, preferably masitinib mesilate, is comprised in a pharmaceutical composition in an amount of at least 50 mg and less than 600 mg, preferably of at least 100 mg and less than 400 mg.

### DEFINITIONS

In the present invention, the following terms have the following meanings:
As disclosed above, the expression **"systemic form of mastocytosis"** as used in the present application, encompasses a subpopulation of patients from the overall mastocytosis patient population. The term **"systemic mastocytosis"** in this invention is to be fully distinguished from the broader terms "mastocytosis" or even "mast cell activation disease", which encompasses a collection of disorders or syndromes characterized by the accumulation of pathological mast cells in potentially any or all organs and tissues and/or the aberrant release of variable subsets of mast cell mediators.

In a first embodiment, **"systemic mastocytosis"** corresponds to the mastocytosis subpopulations of indolent systemic mastocytosis and smoldering systemic mastocytosis, as defined according to the World Health Organization (WHO) diagnostic criteria [Arock M, et al. Eur J Haematol. 2015 Jun;94(6):474-90]. A diagnosis of systemic mastocytosis is made according to the presence of B-('Borderline Benign') findings and C-(`Consider Cytoreduction') findings, which reflect the disease burden (B) and disease aggressiveness (C), respectively. Patients who have systemic mastocytosis without B- or C-findings and without signs of an associated clonal hematologic non-mast cell lineage disease (AHNMD) are diagnosed as **"indolent systemic mastocytosis"** (ISM). Patients who have two or more B-findings, but no C-findings, are diagnosed with **"smoldering systemic mastocytosis"** (SSM), a separate systemic mastocytosis category initially considered a subtype of ISM defined by a huge mass of neoplastic mast cells.

In a second embodiment, **"systemic mastocytosis"** corresponds to the mastocytosis subpopulation as defined according to the AB Science Systemic Mastocytosis (ABSSM) diagnostic criteria, and thus relates to a disease for which any the following four criteria are fulfilled at baseline or before baseline:
- Presence of mast cells in any bone marrow biopsy or aspirate associated with at least one sign of abnormality, wherein said abnormal signs are:
   - More than 1% infiltration of mast cells in bone marrow;
   - Aggregates of more than 15 mast cells in bone marrow that can be described by the following words: nodules, seats, clusters, foci, or granuloma;
   - More than 25% atypical mast cells in a sample of bone marrow;
   - Abnormal mast cells in the sample of bone marrow with microscopic testing that can be described by the following words: spindled, abnormal, atypical, fusiform, dystrophic, pathologic, dysmorphic;
   - Abnormal immunohistochemistry signs: mast cells in bone marrow express CD2 and/or CD25 present;
   - c-Kit point mutation at codon 816 (c-Kit 816) in bone marrow.
- Detection of c-Kit 816 in bone marrow without evidence of mast cells in bone marrow and detection of c-Kit 816 in skin biopsy justifying clonality;
- Presence of mast cells in bone marrow biopsy or aspirate without signs of mast cell abnormality;
- Excess of mast cells detected in digestive organs in addition to excess of mast cells in the skin.

In a third embodiment, **"systemic mastocytosis"** corresponds to the mastocytosis subpopulation as defined according to the Protocol AB06006 version 7.0 (v7) diagnostic criteria and thus relates to a disease for which the following criteria are fulfilled:
- Patient with one of the following documented mastocytosis as per WHO classification:
   - Smoldering Systemic Mastocytosis;
   - Indolent Systemic Mastocytosis; and/or
- Patient with documented mastocytosis and evaluable disease based upon histological criteria including typical infiltrates of mast cells in a multifocal or diffuse pattern in skin and/or bone marrow biopsy.

The term **"indolent form of mastocytosis"** as used in the present application, corresponds to a patient cohort comprised of: smoldering systemic mastocytosis, indolent systemic mastocytosis or cutaneous mastocytosis, each being as defined in the WHO consensus classification system for mastocytosis [Arock M, et al. Eur J Haematol. 2015 Jun;94(6):474-90] .

The term **"aggressive form of mastocytosis"** as used in the present application, corresponds to a patient cohort comprised of: systemic mastocytosis with an associated clonal hematologic non-mast cell lineage disease (SM-AHNMD), aggressive systemic mastocytosis (ASM), mast cell leukemia (MCL), mast cell sarcoma (MCS), and extracutaneous mastocytoma, each being as defined in the WHO consensus classification system for mastocytosis [Arock M, et al. Eur J Haematol. 2015 Jun;94(6):474-90].

The term **"subject"** refers to a mammal, preferably a human. A subject may be a **"patient",** i.e. a warm-blooded animal, more preferably a human, who/which is awaiting the receipt of, or is receiving medical care or was/is/will be the object of a medical procedure, or is monitored for the development of severe systemic mastocytosis.

The terms **"treating"** or **"treatment"** refers to both therapeutic treatment and prophylactic or preventative measures; wherein the object is to prevent or slow down (lessen) severe systemic mastocytosis. Those in need of treatment include those already with severe systemic mastocytosis as well as those prone to have severe systemic mastocytosis or those in whom severe systemic mastocytosis is to be prevented. A subject is successfully "treated" for severe systemic mastocytosis if, after receiving a therapeutic amount of masitinib or a pharmaceutically acceptable salt or solvate according to the present invention, the subject shows observable and/or measurable reduction in or absence of one or more of the following: reduction in the number of pathogenic cells; reduction in the percent of total cells that are pathogenic; and/or relief to some extent, of one or more of the symptoms associated with severe systemic mastocytosis; reduced morbidity and mortality, and improvement in quality of life issues. The above parameters for assessing successful treatment and improvement in the disease are readily measurable by routine procedures familiar to a physician.

The term **"therapeutically effective amount"** means the level or amount of agent that is aimed at, without causing significant negative or adverse side effects to the target, (1) delaying or preventing the onset of severe systemic mastocytosis; (2) slowing down or stopping the progression, aggravation, or deterioration of one or more symptoms of severe systemic mastocytosis; (3) bringing about ameliorations of the symptoms of severe systemic mastocytosis; (4) reducing the severity or incidence of severe systemic mastocytosis; or (5) curing severe systemic mastocytosis. A therapeutically effective amount may be administered prior to the onset of severe systemic mastocytosis, for a prophylactic or preventive action. Alternatively or additionally, the therapeutically effective amount may be administered after initiation of severe systemic mastocytosis, for a therapeutic action or maintenance of a therapeutic action.

The term **"pharmaceutically acceptable carrier or excipient"** refers to an excipient or carrier that does not produce an adverse, allergic or other untoward reaction when administered to a human. It includes any and all solvents, dispersion media, coatings, antibacterial and antifungal agents, isotonic and absorption delaying agents and the like. For human administration, injected preparations should meet sterility, pyrogenicity, general safety and purity standards as required by regulatory offices, such as, for example, FDA Office or EMA.

The term **"solvate"** is used herein to describe a molecular complex comprising the compound (i.e., masitinib) and one or more pharmaceutically acceptable solvent molecules.

The term **"about"** preceding a figure means plus or less 10% of the value of said figure.

### DETAILED DESCRIPTION

The present invention therefore relates to masitinib or a pharmaceutically acceptable salt or solvate thereof for use in the treatment of severe systemic mastocytosis in a human patient, wherein said severe systemic mastocytosis is associated with at least two mast cell mediator release associated handicaps selected from a pruritus score ≥ 9; a number of flushes per week ≥ 8; a Hamilton rating scale for depression (HAMD-17) score ≥ 19; a number of stools per day ≥ 4; a number of micturition per day ≥ 8; and a fatigue (asthenia) score measured by Fatigue Impact Scale (FIS) ≥ 75, and wherein at least two mast cell mediator release associated handicaps are selected from a pruritus score ≥ 9; a number of flushes per week ≥ 8; and a HAMD-17 score ≥ 19, wherein said systemic mastocytosis is smoldering systemic mastocytosis or indolent systemic mastocytosis.

Preferably, a therapeutically effective amount of masitinib or a pharmaceutically acceptable salt or solvate thereof is administered to the subject.

Tyrosine kinases are receptor type or non-receptor type proteins, which transfer the terminal phosphate of ATP to tyrosine residues of proteins thereby activating or inactivating signal transduction pathways. These proteins are known to be involved in many cellular mechanisms, which in case of disruption, lead to disorders such as abnormal cell proliferation and migration as well as inflammation. Within the meaning of the present application, a "tyrosine kinase inhibitor" is thus a drug that inhibits tyrosine kinases, thereby interfering with signaling processes within cells. Blocking such processes can stop the cell growing and dividing.

Pharmaceutically acceptable salts preferably are pharmaceutically acceptable acid addition salts, like for example with inorganic acids, such as hydrochloric acid, sulfuric acid or a phosphoric acid, or with suitable organic carboxylic or sulfonic acids, for example aliphatic mono- or di-carboxylic acids, such as trifluoroacetic acid, acetic acid, propionic acid, glycolic acid, succinic acid, maleic acid, fumaric acid, hydroxymaleic acid, malic acid, tartaric acid, citric acid or oxalic acid, or amino acids such as arginine or lysine, aromatic carboxylic acids, such as benzoic acid, 2-phenoxy-benzoic acid, 2-acetoxy-benzoic acid, salicylic acid, 4-aminosalicylic acid, aromatic-aliphatic carboxylic acids, such as mandelic acid or cinnamic acid, heteroaromatic carboxylic acids, such as nicotinic acid or isonicotinic acid, aliphatic sulfonic acids, such as methane-, ethane- or 2-hydroxyethane-sulfonic, in particular methanesulfonic acid, or aromatic sulfonic acids, for example benzene-, p-toluene- or naphthalene-2-sulfonic acid.

Unless otherwise indicated, references to "mesilate" are used in the present invention to refer to a salt of methane sulfonic acid with a named pharmaceutical substance (such as masitinib). Use of mesilate rather than mesylate is in compliance with the INNM (International nonproprietary names modified) issued by WHO (e.g. World Health Organization (February 2006). International Nonproprietary Names Modified. INN Working Document 05.167/3. WHO.). For example, masitinib or imatinib mesilate mean the methanesulfonic acid salt of masitinib and imatinib, respectively.

### Masitinib as tyrosine kinase inhibitor for use in the present invention

Preferably, "masitinib mesilate" means the orally bioavailable mesilate salt of masitinib - CAS 1048007-93-7 (MsOH); C₂₈H₃₀N₆OS.CH₃SO₃H; MW 594.76:

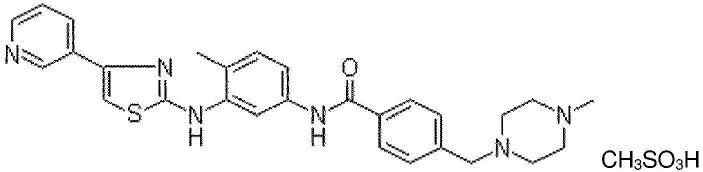

The chemical name for masitinib is 4-(4-methylpiperazin-1-ylmethyl)-N-[4-methyl-3-(4-pyridin-3ylthiazol-2-ylamino) phenyl]benzamide - CAS number 790299-79-5.

Masitinib was described in US 7,423,055 and EP 1 525 200 B1. A detailed procedure for the synthesis of masitinib mesilate is given in WO 2008/098949.

Masitinib mesilate can preferably be used as a c-Kit inhibitor.

In a particular embodiment, masitinib or a pharmaceutically acceptable salt or solvate thereof for use in the treatment of severe systemic mastocytosis according to the invention is an inhibitor of wild-type c-Kit and/or Lyn or Fyn kinase activity. Stem cell factor (SCF), the ligand of the c-Kit receptor, was indeed shown to be a major growth factor for mast cell survival, proliferation, differentiation, adhesion and degranulation processes [Reber et al., Eur J Pharmacol 2008;533:327-340], with SCF-dependent activation of c-Kit critical for mast cell homeostasis and function. The symptoms of severe systemic mastocytosis are in particular resulting from the inappropriate or enhanced release of mast cells mediators. In a particular embodiment, masitinib or a pharmaceutically acceptable salt or solvate thereof for use according to the present invention is thus capable of inhibiting specifically the SCF/c-Kit pathway. Similarly, Lyn and Fyn kinases were shown to play the role of key components of the transduction pathway leading to IgE induced degranulation [Gilfillan & Tkaczyk, 2006, Nat Rev Immunol, 6:218-230; Gilfillan et al., 2009, Immunological Reviews, 228:149-169]. Moreover, it has been shown that Lyn is an important signaling molecule that contributes to growth of neoplastic mast cells in advanced systemic mastocytosis [Gleixner et al. Blood. 2011 Aug 18;118(7):1885-98]. In a particular embodiment, masitinib or a pharmaceutically acceptable salt or solvate thereof for use in the treatment of severe systemic mastocytosis according to the present invention is thus capable of inhibiting specifically the activity Lyn and Fyn kinases.

Masitinib is a small molecule selectively inhibiting specific tyrosine kinases such as c-Kit, PDGFR, Lyn, Fyn and to a lesser extent the fibroblast growth factor receptor 3 (FGFR3), without inhibiting, at therapeutic doses, kinases associated with known toxicities (i.e. those tyrosine kinases or tyrosine kinase receptors attributed to possible tyrosine kinase inhibitor cardiac toxicity, including ABL, KDR and Src) [Dubreuil et al., 2009, PLoS ONE 2009.4(9):e7258]. Masitinib's strong inhibitory effect on wild-type and juxtamembrane-mutated c-Kit receptors, results in cell cycle arrest and apoptosis of cell lines dependent on c-Kit signaling [Dubreuil et al., 2009, PLoS ONE, 4(9):e7258]. Stem cell factor, the ligand of the c-Kit receptor, is a critical growth factor for mast cells; thus, masitinib is an effective antimastocyte, exerting a direct antiproliferative and pro-apoptotic action on mast cells through its inhibition of c-Kit signaling. Moreover, *in vitro,* masitinib demonstrated greater activity and selectivity against c-Kit than imatinib, inhibiting recombinant human wild-type c-Kit with a half inhibitory concentration (IC₅₀) of 200 ± 40 nM and blocking stem cell factor-induced proliferation and c-Kit tyrosine phosphorylation with an IC₅₀ of 150 ± 80 nM in Ba/F3 cells expressing human or mouse wild-type c-Kit. In contrast, masitinib only weakly inhibited the proliferation of Ba/F3 cells expressing the D816V c-Kit mutation with an IC₅₀ of 5.0 ± 2.0 µM, and by consequence is inactive, at therapeutically viable doses, against the D816V mutation of c-Kit.

In addition to its antiproliferative properties, masitinib can also regulate the activation of mast cells through its targeting of Lyn and Fyn, key components of the transduction pathway leading to IgE induced degranulation.

### Treatment of severe systemic mastocytosis with masitinib

Molecules able to inhibit the survival and/or activation of mast cells may be able to control the symptoms and progression of severe systemic mastocytosis. We consider that a tyrosine kinase inhibitor, notably as defined above, especially masitinib, through its inhibition of mast cell proliferation and activation, is fulfilling this role in the treatment of severe systemic mastocytosis, via reducing the overall mast cell burden and inhibiting the global activity of mast cells.

It would seem without willing to be bound by a theory, surprisingly that a tyrosine kinase inhibitor, notably as defined above, especially masitinib, could also be of further therapeutic benefit against severe systemic mastocytosis by inhibiting mast cell activity via inhibition of Lyn and Fyn. This is significant as it represents a mechanism of action that is independent from the c-Kit signaling pathway or survival of mast cells. It follows that the subsequent decrease in mast cell activity would lead to a lessening of mast cell mediator release symptoms and severe systemic mastocytosis related handicap. In addition, a reduction in release of various chemoattractants associated with mast cell migration will lessen the rate of mast recruitment and accumulation, further lessening the symptoms of severe systemic mastocytosis patients dampening their cascade effect. For example, SCF is a chemotactic factor for mast cells, with mast cells themselves possessing the capacity to synthesize, store and release SCF. Thus, expression of SCF is increased in the activation of mast cells, with subsequent migration of other mast cells towards this source of SCF, cumulating in mast cell accumulation. If the mast cell mediator release encountered in severe systemic mastocytosis is due to an intrinsic defect that lowers the activation threshold of mast cells, then masitinib's inhibition of activity would help compensate or restore normal function, with respect to mediator hypersecretion and release of the mast cell chemoattractants, such as SCF.

A reduction of mast cell activity via the inhibitory action of masitinib on c-Kit, Lyn and Fyn tyrosine kinase activity, may thus impact the overall mast cell burden and inflammatory cascade as well as the threshold of mast cell activation and migration/recruitment of mast cells. Unexpectedly, without willing to be bound by a theory, it is through this multifaceted mechanism of action that the use of masitinib according to the invention can elicit a response in severe systemic mastocytosis patients.

In relation to the present invention, severe systemic mastocytosis in a human patient is defined by the presence of severe mast cell mediator release associated handicap(s) in said human patient. In other words, a human patient suffering from severe systemic mastocytosis is a human patient suffering from systemic mastocytosis with severe handicap(s) associated with mast cell mediator release.

In relation to the present invention, the term "treatment" (and its various grammatical forms) refers to preventing, curing, reversing, attenuating, alleviating, minimizing, suppressing or halting the deleterious effects of a disease state, disease progression, disease causative agent (e.g., bacteria or viruses) or other abnormal condition. For example, treatment may involve alleviating a symptom (i.e., not necessary all symptoms) of a disease or attenuating the progression of a disease.

The inventors have surprisingly shown that masitinib provides therapeutic benefit to a highly distinct subpopulation of mastocytosis patients, with the severity of mast cell mediator release associated handicaps serving as an independent predictor factor for treatment efficacy, in particular the severe handicaps of pruritus and flush (see Examples 2 and 3).

A randomized placebo-controlled phase 3 study (AB06006) was conducted based on the aforementioned preliminary clinical data to determine the efficacy and safety of masitinib compared with placebo as first-line therapy in patients with cutaneous mastocytosis, smoldering systemic mastocytosis, or indolent systemic mastocytosis with handicap (see ClinicalTrials.gov NCT00814073) (see Example 1). Patient handicapped status was originally defined as "pruritus score ≥ 6, number of flushes per week ≥ 7, Hamilton rating scale (depression) ≥ 10, number of stools per day ≥ 4, number of mictions per day ≥ 8, Fatigue Impact Scale total score (asthenia) ≥ 40". These thresholds for mast cell mediator release associated handicaps describe mild to moderate handicaps; for example, the Hamilton rating scale (depression) categories are defined according to the scoring manual as: 0-7 normal, 8-13 mild depression, 14-18 moderate depression, 19-22 severe depression, ≥ 23 very severe depression. It was anticipated that the principal mechanism of action responsible for any improvement in masitinib treated patients would be a masitinib-induced inhibition of mast cell function as opposed to a predominantly cytoreductive mechanism of action. It was hypothesized therefore, that the expected patient population to benefit from masitinib treatment would include all those patients with indolent forms of mastocytosis (including smoldering systemic, indolent systemic and cutaneous mastocytosis), regardless of severity of mast cell mediator release associated handicap (i.e. mild, moderate or severe handicap), as opposed to the aggressive forms of mastocytosis (which are selected from ASM, SM-AHNMD, MCL, MCS, and extracutaneous mastocytoma, each being as defined in the WHO consensus classification system for mastocytosis).

The inventors have surprisingly shown that in a subpopulation of severe systemic mastocytosis defined with restricted inclusion criteria, masitinib does generate a clinical advantage compared with placebo. This subpopulation is defined as follows:
- Patient with documented systemic mastocytosis as defined according to either the World Health Organization (WHO) diagnostic criteria, or the AB Science Systemic Mastocytosis (ABSSM) diagnostic criteria, or the Protocol AB06006 v7 diagnostic criteria; as defined hereinabove;
- Severe mast cell mediator release associated handicap defined as the following handicaps, including at least one, preferably 2, 3 or 4 among pruritus, flushes, depression, or fatigue; and including at least one, preferably 2 among pruritus or flushes:
   - Pruritus score ≥ 9;
   - Number of flushes per week ≥ 8;
   - Hamilton rating scale for depression (HAMD-17) score ≥ 19;
   - Number of stools per day ≥ 4;
   - Number of micturition per day ≥ 8;
   - Fatigue Impact Scale total score (asthenia) ≥ 75.

The inventors have surprisingly shown therefore that a distinct subpopulation responds to masitinib treatment, with said distinction being made according to the severity (degree) of mast cell mediator release associated handicap (see Example 3). These findings are without precedent because the predictive therapeutic significance of mast cell mediator release associated handicap severity in mastocytosis patients was previously unknown, in particular the predictive therapeutic significance of severe pruritus and severe flush handicaps. This outcome could not have been predicted by teachings from the prior-art.

Proof of concept that masitinib can generate a clinically relevant therapeutic benefit in the subpopulation of severe systemic mastocytosis is supported via post-hoc analysis of data from two phase II clinical trials conducted in a relevant patient population (see Example 2). For patients treated with masitinib a response rate (≥ 75% improvement from baseline) of 25.0% and 23.5% was reported after 12 and 24 weeks of treatment, respectively, based on a pooled cohort that closely simulated the selected subpopulation of severe systemic mastocytosis and response criteria from study AB06006 (see Example 1). Further it was surprisingly revealed that masitinib provides greatest therapeutic benefit in the severe mast cell mediator release associated handicaps of pruritus and flushes, i.e. pruritus and flushes are the variables of greatest predictive power. In contrast masitinib provided only minor response rates in the severe mast cell mediator release associated handicaps of depression and fatigue. This contrast in predictive power is maintained when pairing variables of [flushes or pruritus] as compared with [Hamilton or FIS], with cumulative response rates of approximately 40% versus 10%, respectively. Additionally, it was shown that those systemic mastocytosis patients having handicaps of both pruritus and flushes experienced an unprecedentedly high response rate of 50% after 24 weeks of masitinib treatment.

In a particular embodiment, masitinib or a pharmaceutically acceptable salt or solvate thereof for use according to the invention treats a severe systemic mastocytosis patient, wherein said systemic form of mastocytosis is defined according to the WHO diagnostic criteria for smoldering systemic mastocytosis or indolent systemic mastocytosis, or by the ABSSM diagnostic criteria, or by the Protocol AB06006 v7 diagnostic criteria, as defined hereinabove.

As described above, it indeed appears that patients suffering from systemic mastocytosis often experience symptoms resulting from the constitutive activation of mast cells and release of their mediators. Collectively, these are referred to as "mast cell mediator release symptoms". These symptoms may in particular comprise asthenia (fatigue), pruritus, flushing, micturition frequency (pollakiuria), stool frequency (diarrhea), and depression.

The present invention was advantageously shown to provide a significant beneficial effect on systemic mastocytosis patients with severe mast cell mediator release associated handicap.

In one embodiment said severe handicap comprises at least 2 or 3, of pruritus, flushes or depression, of which preferably at least one should be pruritus or flushes, and wherein if present said handicaps are considered as being severe on an appropriate scale of said handicap, having the following scores: pruritus score ≥ 9; number of flushes per week ≥ 8; depression measured by the Hamilton rating scale ≥ 19.

In one embodiment, said severe systemic mastocytosis is associated with at least a pruritus score ≥ 9 and a number of flushes per week ≥ 8; a pruritus score ≥ 9 and a Hamilton rating scale for depression (HAMD-17) score ≥ 19; or a number of flushes per week ≥ 8 and a Hamilton rating scale for depression (HAMD-17) score ≥ 19.

In one embodiment, said severe systemic mastocytosis is associated with at least a pruritus score ≥ 9, a number of flushes per week ≥ 8 and a Hamilton rating scale for depression (HAMD-17) score ≥ 19; a pruritus score ≥ 9, a number of flushes per week ≥ 8 and a fatigue score (asthenia) measured by Fatigue Impact Scale ≥ 75; or a number of flushes per week ≥ 8, a Hamilton rating scale for depression (HAMD-17) score ≥ 19 and a fatigue score (asthenia) measured by Fatigue Impact Scale ≥ 75.

In one embodiment, said severe systemic mastocytosis is associated with at least a pruritus score ≥ 9; a number of flushes per week ≥ 8, a Hamilton rating scale for depression (HAMD-17) score ≥ 19 and a fatigue score (asthenia) measured by Fatigue Impact Scale ≥ 75.

In a particular embodiment, masitinib or a pharmaceutically acceptable salt or solvate thereof for use according to the present invention in particular advantageously demonstrated a treatment response as a ≥ 75% improvement of the handicap from baseline. The level of response obtained with masitinib or a pharmaceutically acceptable salt or solvate thereof for use according to the invention thus represents a highly clinically relevant improvement.

In one embodiment, the subject is not a mast cell activation syndrome (MCAS) patient.

The expression "mast cell activation syndrome" (MCAS), as used in the present application, encompasses a collection of clinical signs and symptoms resulting from the inappropriate activation of mast cells, wherein no proliferation or otherwise accumulation of mast cells is observed. According to an embodiment, MCAS corresponds to the syndrome defined by the Molderings criteria, or the Valent diagnostic criteria, or the Akin diagnostic criteria, as defined below.

In a first embodiment, MCAS is defined according to Molderings et al. (Molderings et al., Journal of Hematology & Oncology, 2011, 4:10), and thus relates to a syndrome for which both hereinafter major criteria or the second hereinafter major criterion and at least one hereinafter minor criterion are fulfilled:
- Major criteria:
   ∘ Multifocal or disseminated dense infiltrates of mast cells in bone marrow biopsies and/or in sections of other extracutaneous organ(s), e.g. gastrointestinal tract biopsies; CD117-, tryptase- and CD25-stained;
   ∘ Unique constellation of clinical complaints as a result of a pathologically increased mast cell activity (mast cell mediator release syndrome).
- Minor criteria:
   ∘ Mast cells in bone marrow or other extracutaneous organ(s) show an abnormal morphology (> 25%) in bone marrow smears or in histologies;
   ∘ Mast cells in bone marrow express CD2 and/or CD25;
   ∘ Detection of genetic changes in mast cells from blood, bone marrow or extracutaneous organs for which an impact on the state of activity of affected mast cells in terms of an increased activity has been proved;
   ∘ Evidence of a pathologically increased release of mast cell mediators by determination of the content of: tryptase in blood, N-methylhistamine in urine, heparin in blood, chromogranin A in blood, other mast cell-specific mediators (e.g. leukotrienes, prostaglandin D₂).

In a second embodiment, the term MCAS is defined by the Valent diagnostic criteria (Valent et al, Int Arch Allergy Immunol 2012, 157:215-225), and thus relates to a syndrome for which the following criteria are fulfilled:
1. Typical clinical signs and symptoms, including: flushing, pruritus, urticaria, angioedema, nasal congestion, nasal pruritus, wheezing, throat swelling, headache, hypotension, and diarrhea;
2. Substantial and transient increase in a mast cell (MC)-derived mediator in biological fluids [preferably serum total tryptase, but also histamine/histamine metabolites and prostaglandin D2 (PGD2)/PGD2 urinary metabolites] during or shortly after the acute event compared to a baseline level recorded either before the acute event or at least 24 h after all clinical signs and symptoms of the event have completely resolved;
3. An objective major response of clinical symptoms to agents that attenuate the production or activity of MC-derived mediators.

According to the definition of Valent et al, all three criteria should be met to define MCAS. However, in some circumstances, a patient may not respond to a drug and may even require intensive care and epinephrine administration. In such a patient, the condition may still be considered as MCAS if typical symptoms (1) and an increase in MC mediators (2) are present, and a primary underlying MC disease (e.g. SM) or an underlying IgE-mediated disease (e.g. allergy) is known.

In a third embodiment, the term MCAS is defined by the Akin diagnostic criteria (Akin et al., J Allergy Clin Immunol, 2010, 126(6):1099-1104), and thus relates to a syndrome for which the following criteria are fulfilled:
- Episodic symptoms consistent with mast cell mediator release affecting two or more organ systems evidenced as follows:
   ∘ skin: urticarial, angioedema, flushing;
   ∘ gastrointestinal: nausea, vomiting, diarrhea, abdominal cramping;
   ∘ cardiovascular: hypotensive syncope or near syncope, tachycardia;
   ∘ respiratory: wheezing;
   ∘ naso-ocular: conjunctival injection, pruritus, nasal stuffiness;
- A decrease in the frequency or severity, or resolution of symptoms with antimediator therapy: H1 and H2 histamine receptor antagonists, anti-leukotriene medications (cysLT receptor blockers or 5-LO inhibitor), or mast cell stabilizers (cromolyn sodium);
- Evidence of an elevation in a validated urinary or serum marker of mast cell activation: documentation of elevation of the marker above the patient's baseline during a symptomatic period on at least two occasions; or if baseline tryptase levels are persistently > 15 ng, documentation of elevation of the tryptase above baseline in one occasion. Total serum tryptase is recommended as the markers of choice; less specific (also from basophils) 24 hours urine histamine metabolites or 11-beta-prostaglandin F2;
- Primary (clonal) and secondary disorders of mast cell activation ruled out. Primary disorders of mast cell activation include: anaphylaxis with an associated clonal mast cell disorder, and monoclonal mast cell activation syndrome (MMAS). Secondary disorders of mast cell activation include: allergic disorders, mast cell activation associated with chronic inflammatory or neoplastic disorders, physical urticarias, chronic autoimmune urticaria.

### Dosage regimen

Regarding best dosage regimen, masitinib or a pharmaceutically acceptable salt or solvate thereof for use according to the invention is to be administered at a daily dose of between about 1.5 to 9.0 mg/kg/day; for example, 1.5, 3.0, 4.5, 6.0, 7.5, or 9.0 mg/kg, more preferably 3.0, 4.5 or 6 mg/kg/day (mg per kg bodyweight per day).

Nonetheless said masitinib or a pharmaceutically acceptable salt or solvate thereof can be dose escalated by increments of about 1.5 mg/kg/day in low responder patients to reach a maximum of about 9.0 mg/kg/day, more preferably 6 mg/kg/day.

In one embodiment dose escalation of said masitinib or a pharmaceutically acceptable salt or solvate thereof occurs at any time-point after at least 4 weeks after the initial dose has been administered and prior to 26 weeks after the initial dose has been administered; for example at week-4, week-8, week-12, week-16, week-20, or week-24.

Each dose escalation is subjected to toxicity controls, including : previous 4-week treatment period at a constant dose of study treatment and no suspected severe adverse event was reported and no suspected adverse event led to treatment interruption and no suspected adverse event is ongoing at the time of the dose increase, regardless of its severity. In the absence of any of the above mentioned toxicity events, the predefined dose escalation may occur. In the case of an ongoing non-severe suspected adverse event at the time of the dose escalation or treatment interruption without dose reduction at time of treatment resumption, any dose increase is delayed until after an additional 4-week treatment period. No dose escalation will be authorized for patients who have had a dose reduction for safety reasons.

In one embodiment said masitinib or a pharmaceutically acceptable salt or solvate thereof is to be initially administered per os, preferably in two daily intakes, at a dose of 3 mg/kg/day during 4 weeks, then 4.5 mg/kg/day during 4 weeks, and then 6 mg/kg/day thereafter.

In another embodiment said masitinib or a pharmaceutically acceptable salt or solvate thereof is to be initially administered at a dose of 4.5 mg/kg/day during 12 weeks, and then 6 mg/kg/day thereafter.

Any dose indicated herein refers to the amount of active ingredient as such, not to its salt form.

Given that the masitinib dose in mg/kg/day used in the described dose regimens refers to the amount of active ingredient masitinib, compositional variations of a pharmaceutically acceptable salt of masitinib mesilate will not change the said dose regimens.

In a particular embodiment, masitinib or a pharmaceutically acceptable salt or solvate thereof may further be administered via different routes of administration but oral administration is preferred. Thus, in still another preferred embodiment, in the use above, masitinib or salts or solvates thereof, is to be administered orally; preferably twice a day for long term period such as over more than 6 months, preferably more than 12 months. Masitinib or a pharmaceutically acceptable salt or solvate thereof can be administered in the form of 100 and 200 mg tablets.

According to a particular embodiment, the pharmaceutical composition or medicament for use in treating severe systemic mastocytosis according to the invention is thus an oral composition.

As is known to the person skilled in the art, various forms of excipients can be used adapted to the mode of administration and some of them can promote the effectiveness of the active molecule, e.g. by promoting a release profile rendering this active molecule overall more effective for the treatment desired.

Pharmaceutical compositions, medicaments or compositions for use according to the invention are thus able to be administered in various forms, more specially for example in an injectable, pulverizable or ingestible form, for example via the intramuscular, intravenous, subcutaneous, intradermal, oral, topical, rectal, vaginal, ophthalmic, nasal, transdermal or parenteral route. A preferred route is oral administration.

Such medicament or pharmaceutical composition can take the form of a medicament or pharmaceutical composition adapted for oral administration, which can be formulated using pharmaceutically acceptable carriers well known in the art in suitable dosages. Such carriers enable the pharmaceutical compositions to be formulated as tablets, pills, dragees, capsules, liquids, gels, syrups, slurries, suspensions, and the like, for ingestion by the patient. In addition to the active ingredients, these pharmaceutical compositions may contain suitable pharmaceutically-acceptable carriers comprising excipients and auxiliaries which facilitate processing of the active compounds into preparations which can be used pharmaceutically. Further details on techniques for formulation and administration may be found in the latest edition of Remington's Pharmaceutical Sciences (Maack Publishing Co., Easton, Pa.).

### EXAMPLES

The present invention is further illustrated by the following examples.

### Example 1

### Study AB06006 design

Study AB06006 was a prospective, multicenter, randomized, double blind, placebo-controlled, 2-parallel group with a randomization 1:1, phase 3 study to compare efficacy and safety of masitinib at 6 mg/kg/day to placebo in treatment of patients with documented smoldering systemic mastocytosis, indolent systemic mastocytosis or cutaneous mastocytosis, with (mild to moderate) handicap. The trial lasted 24 weeks with possible extension.

In accordance with protocol amendment version 7.0 (v7), the study population was amended to a more restricted subpopulation of mastocytosis. The objective therefore became to compare efficacy and safety of masitinib at 6 mg/kg/day to placebo in treatment of patients with systemic mastocytosis with severe handicap (patients with severe systemic mastocytosis). The trial lasted 24 weeks with possible extension.

### Inclusion of patients having systemic mastocytosis

Eligible patients according to the protocol AB06006 v7 diagnostic criteria thus relate to a disease for which the following criteria are fulfilled:
1. Patient with one of the following documented mastocytosis as per WHO classification:
   a. Smoldering Systemic Mastocytosis;
   b. Indolent Systemic Mastocytosis; and/or
2. Patient with documented mastocytosis and evaluable disease based upon histological criteria including typical infiltrates of mast cells in a multifocal or diffuse pattern in skin and/or bone marrow biopsy.

The former inclusion criterion was applied to include non-aggressive forms of systemic mastocytosis according to WHO classification, while the latter selected patients matching those from the masitinib phase 2 trials, for which this is a confirmatory study. To ensure consistency in the Investigator's application of diagnostic criteria it was necessary to develop a new definition of systemic mastocytosis respecting criteria 1 and 2 of the protocol. To be noted, criterion 2 allows several possibilities for the combination of organs (bone marrow, skin, digestive organs, etc.) and allows for a diagnosis of systemic mastocytosis for patients who have mastocytes in bone marrow without any abnormality.

The resultant diagnostic criteria were referred to as the 'AB Science Systemic Mastocytosis (ABSSM) criteria', as described hereinabove. Thus, a diagnosis of systemic mastocytosis is verified if any the following four criteria are fulfilled at baseline or before baseline:
- Presence of mast cells in any bone marrow biopsy or aspirate associated with at least one sign of abnormality, wherein said abnormal signs are:
   - More than 1% infiltration of mast cells in bone marrow;
   - Aggregates of more than 15 mast cells in bone marrow that can be described by the following words: nodules, seats, clusters, foci, or granuloma;
   - More than 25% atypical mast cells in a sample of bone marrow;
   - Abnormal mast cells in the sample of bone marrow with microscopic testing that can be described by the following words: spindled, abnormal, atypical, fusiform, dystrophic, pathologic, dysmorphic;
   - Abnormal immunohistochemistry signs: mast cells in bone marrow express CD2 and/or CD25 present;
   - c-Kit point mutation at codon 816 (c-Kit 816) in bone marrow.
- Detection of c-Kit 816 in bone marrow without evidence of mast cells in bone marrow and detection of c-Kit 816 in skin biopsy justifying clonality;
- Presence of mast cells in bone marrow biopsy or aspirate without signs of mast cell abnormality;
- Excess of mast cells detected in digestive organs in addition to excess of mast cells in the skin.

The phase 3 study was designed to evaluate masitinib efficacy and safety in severe systemic mastocytosis patients, with or without D816V mutation of c-Kit

### Severe systemic mastocytosis

Severe systemic mastocytosis was defined as the presence of at least two of the six following mast cell mediator release associated handicaps: a pruritus score ≥ 9; a number of flushes per week ≥ 8; a Hamilton rating scale for depression (HAMD-17) score ≥ 19; a number of stools per day ≥ 4; a number of micturition per day ≥ 8; a fatigue score (asthenia) measured by Fatigue Impact Scale ≥ 75, wherein at least one handicap is selected among pruritus, flushes, depression and fatigue (asthenia).

To be noted, for the purpose of efficacy analysis a patient was also classified as severe if they suffered from at least one of the main handicaps constituting the primary endpoint: Pruritus score ≥ 9; number of flushes per week ≥ 8; Hamilton rating scale for depression (HAMD-17) score ≥ 19; Fatigue Impact Scale total score (asthenia) ≥ 75. The criterion of "at least two of the following handicaps" including handicaps on stools and micturition, was relaxed to "at least one of the main handicaps" (i.e. pruritus, flushes, depression and fatigue).

Patients enrolled in the phase 3 study had between one and four of the following severe mastocytosis related symptoms at baseline:
- Pruritus score ≥ 9;
- Number of flushes per week ≥ 8;
- Depression measured by the Hamilton rating scale (HAMD-17) score ≥ 19;
- Asthenia measured by the Fatigue Impact Scale total score ≥75.

The study enrolled 135 patients with severe systemic mastocytosis.

Patients with systemic mastocytosis with severe handicap were randomized in two treatment groups including: Group 1: patients received masitinib 6 mg/kg/day, and Group 2: patients received placebo. The two treatment groups were defined such as to equally balance handicap/scores at baseline regarding pruritus, flushes, depression and fatigue, which might influence the study outcome. The randomization procedures included a minimization process aimed at reducing any difference in the distribution of the handicaps/scores at baseline and country in patients with documented smoldering or indolent systemic mastocytosis with severe handicap.

Patients with one of the following documented mastocytosis as per WHO classification were excluded from the study: cutaneous mastocytosis; not documented smoldering systemic mastocytosis or indolent systemic mastocytosis; systemic mastocytosis with an associated clonal hematologic non mast cell lineage disease (SM-AHNMD); mast cell leukemia (MCL); or aggressive systemic mastocytosis (ASM).

### Analysis datasets

Intention-To-Treat (ITT) dataset - The ITT population was defined as all patients randomized presenting a documented systemic mastocytosis with severe handicap as defined above (and as documented also by protocol version 7.0). Patients were classified according to the treatment-arm to which they have been randomized, irrespective of the actual treatment received.

Modified Intent-To-Treat (mITT) dataset - The mITT population included all ITT patients with severe systemic mastocytosis who took at least one dose of study treatment (masitinib/placebo).

Per Protocol (PP) dataset - The PP data set consisted of all patients of the mITT data set without any major protocol deviation. This was the set of patients who participated in the study as intended. Patients terminating the study prematurely were included in the PP data set provided that there was no protocol deviation. Before locking the data base, the precise reasons for excluding patients from the PP data set were fully defined and documented by the Data Review Committee. Protocol deviations were defined as: inclusion and non-inclusion criteria were not met, intake of forbidden medication, non-respect of visit dates, missing value for main criterion without premature termination, non-respect of protocol design, any other deviations during the course of the study.

Safety population - The safety population consists of all patients presenting a documented systemic mastocytosis with severe handicap as defined by protocol version 7.0 who took at least one dose of study medication (masitinib or placebo).

### Statistical Methods

Efficacy: Handicaps are defined as:
- Main handicaps: pruritus score ≥ 9, number of flushes per week ≥ 8, HAMD-17 score ≥ 19, Fatigue Impact Scale ≥ 75;
- Other handicaps: micturition ≥ 8, stools ≥ 4.

Response on a handicap is defined as an improvement ≥ 75% from baseline for pruritus, flushes, Hamilton, and fatigue.

### Primary analysis

The primary objective of the phase 3 study was to detect a statistically significant difference between masitinib (plus concomitant symptomatic treatments) and placebo (plus concomitant symptomatic treatments) in cumulative response on four severe symptoms, referred to also as handicaps.

Cumulative response by patient*handicap - For all the patients, the response at each study visit (5 visits from week 8 to week 24) will be calculated on each handicap present at baseline (among pruritus, flushes, Hamilton and FIS) as defined above. Thus, from 5 to 20 responses will be calculated per patient: 5 if the patient presents only 1 handicap at baseline corresponding to the 5 visits, and 20 if the patient presents with 4 handicaps at baseline corresponding to the 4 handicaps times 5 visits.

Primary analysis (referred to as "4H75% response") was based on the comparison between masitinib and placebo in the number of actual responses between week 8 and week 24 divided by the total number of possible responses over the same treatment period. At each patient evaluation between weeks 8 and 24, each of the above four severe symptoms was evaluated. An improvement ≥ 75% with respect to baseline in one symptom represented one positive treatment response.

For qualitative binary variables, such as a response (yes/no), main analysis was performed with the Missing Data equal to Failure (MDF) method, and sensitivity analysis will be performed on Observed Cases (OC, no replacement of the missing data) The MDF method is defined as follows:
▪ Replacement of missing value for patients withdrawn from the study:
   - Case 1: discontinuation for toxicity, lack of efficacy, or unknown reason. In this case, missing data was imputed as failure (missing = failure as primary analysis);
   - Case 2: discontinuation for a documented reason excluding toxicity or lack of efficacy. In this case, non-observed values can be considered as Missing At Random (MAR), and no imputation will be done (Observed Cases).
▪ Replacement of missing value for patients present at the visit: If data was missing despite the patient being present at the visit, said missing data was not replaced.

### Secondary analysis

Secondary analyses were based on the following endpoints:
- Cumulative 75%-response rate for pruritus (1H75% response);
- Cumulative 75% response rate on the handicaps of pruritus or flushes (2H75% response);
- Cumulative 75% response rate on the handicaps of pruritus or flushes or depression (3H75% response);
- Mean change in tryptase level relative to baseline at week 24, in patients with baseline level ≥20 µg/L.

The symptoms of pruritus and flush are well-recognized to be associated with mast cell activation in mastocytosis.

Depression is a symptom that has an important impact on quality of life of patients suffering from mastocytosis, and is therefore of high clinical relevance.

Tryptase is a biological product released by mast cells and an established marker of mast cell burden and activity.

### Cumulative response on pruritus among patients with the handicap at baseline:

Cumulative response is calculated for pruritus because pruritus is considered as the most objective and representative measure in mastocytosis benefiting from a validated measure. For the patients presenting the handicap at baseline (i.e. score ≥ 9), the response at each study visit (5 visits from week 8 to week 24) will be calculated as defined above. Thus, 5 responses will be calculated by the patient for each secondary variable listed. Replacement of missing data was performed via the Missing Data equal to Failure (MDF) method for patients based on the rules described hereinbefore.

Cumulative response on pruritus and/or flushes among patients with these handicaps at baseline: Cumulative response is calculated for pruritus and/or flushes handicaps because these are direct markers of mast cell activity and therefore the most relevant symptoms of mastocytosis. Handicap at baseline is defined as: pruritus score ≥ 9 and flushes per week ≥ 8. Response is defined as an improvement with respect to the baseline values >75%. For all patients, the response at each study visit (5 visits from Week-8 to Week-24) will be calculated on each handicap present at baseline (among pruritus and flushes), as defined above. Thus, from 5 to 10 responses will be calculated per patient: 5 responses if the patient presents only 1 handicap at baseline (corresponding to the 5 visits) and 10 responses if the patient presents both handicaps at baseline (corresponding to the 2 handicaps times the 5 visits). Replacement of missing data was performed via the Missing Data equal to Failure (MDF) method for patients based on the rules described hereinbefore.

### Example 2

Proof of concept that masitinib can generate a clinically relevant therapeutic benefit in the subpopulation of severe systemic mastocytosis is supported via post-hoc analysis of data from two phase 2 clinical trials conducted in a relevant patient population. The AB04010 phase 2 study was conducted in patients with indolent forms of mastocytosis with handicap and not bearing activating point mutations in the phosphotransferase domain of c-Kit such as the main mutation Asp-816-Val. The AB06013 phase 2 study was conducted in patients with mastocytosis with handicap and bearing activating point mutations in the phosphotransferase domain of c-Kit such as the main mutation Asp-816-Val.

### Subpopulation of severe systemic mastocytosis (at least one severe mast cell mediator release associated handicap at baseline from among pruritus, flush frequency, fatigue, or depression)

In one scenario, among those systemic mastocytosis patients from these pooled studies, 28 patients met the criteria for severe handicap at baseline, as defined as having at least one handicap selected from pruritus, flushes, fatigue or depression (defined respectively as: pruritus score >9, flushes frequency per week ≥8, FIS ≥ 75, or Hamilton score ≥19) with a total of 53 severe baseline handicaps reported (note that a given patient may present with more than one handicap). Assessment of treatment response on a handicap was defined as a ≥ 75% improvement from baseline on at least one handicap among pruritus, flushes, fatigue or depression. Results for overall response rates as well as relative changes in individual handicaps and response rates are presented in Table 1.

Overall, at week 12, there was a response rate of 28.3%. A significant improvement was observed for the variables of flushes frequency (p<0.001), pruritus (p=0.002) and FIS score (p=0.004), with an improving trend in the Hamilton score (p=0.063). At week 24, there was an overall response rate of 23.5%. The slight decrease in response rate between week 12 to week 24 was in part due to patients deciding not to enter the studies extension phases. However, statistically significant improvement was maintained over this time for the variables of flushes frequency (p<0.001), pruritus score (p=0.002) and FIS score (p=0.004) with again an improving trend in the Hamilton score (p=0.063).

Considering cumulative response analysis, i.e. the sum of actual responses between weeks 8 and 24 (3 time-points: W8, W12, and W24) divided by the total number of possible responses over the same treatment period, the response rate was 26.1% (see Table 2).

**Table 1. Masitinib treatment effect in pooled (AB06013 and AB04010 mastocytosis studies) cohort simulating a subpopulation of severe systemic mastocytosis (at least one severe mast cell mediator release associated handicap at baseline from among pruritus, flush frequency, fatigue, or depression) - Overall response and individual handicap change.**

| | | Week 12 | Week 24 |
|---|---|---|---|
| Overall response ≥ 75%*^{†} | | 25.0% | 23.5% |
| | | | |
| Number of flushes ≥ 8 | Relative change** | -64.6% (30.8) | -75.3% (30.0) |
| | p value | <0.001 | <0.001 |
| | | | |
| Pruritus score ≥ 9 | Relative change** | -37.6% (43.3) | -34.5% (42.9) |
| | p value | 0.002 | 0.003 |
| | | | |
| FIS score ≥ 75 | Relative change** | -27.2% (21.7) | -27.6% (25.1) |
| | p value | <0.001 | <0.001 |
| | | | |
| Hamilton score ≥ 19 | Relative change** | -40.5% (37.1) | -42.0% (36.1) |
| | p value | 0.063 | 0.063 |

| | | | |
|---|---|---|---|
| * Response rate missing data considered as failure. † Response defined as a ≥ 75% improvement from baseline on at least one handicap among pruritus, flushes, fatigue or depression. ** Mean relative change (%) with respect to baseline (SD), Last Observation Carried Forward. | | | |

### Pruritus and flushes are the variables of greatest predictive power

An analysis of response rates associated with specific handicap variables, as opposed to overall response rates, has unexpectedly and surprisingly revealed that masitinib provides greatest therapeutic benefit in the severe mast cell mediator release associated handicaps of pruritus and flushes (Table 2). An equally unexpected and surprising finding was that masitinib provided only minor response rates in the severe mast cell mediator release associated handicaps of depression, fatigue, micturition frequency (pollakiuria), and stool frequency (diarrhea), making them vulnerable to masking due placebo-effect, which is expected to be high in this indication (i.e. any treatment-effect is indiscernible with respect to placebo treatment). This contrast in predictive power is maintained when pairing variables of [flushes or pruritus] when compared with [Hamilton or FIS], with cumulative response rates of approximately 40% versus 10%, respectively (see Table 2).

**Table 2. Breakdown of individual handicap response rates. Masitinib treatment effect in pooled (AB06013 and AB04010 mastocytosis studies) cohort simulating a subpopulation of severe systemic mastocytosis (severe mast cell mediator release associated handicap at baseline from among pruritus, flush frequency, fatigue, or depression) - Breakdown of individual handicap response rates.**

| | **Cumulative response rate (≥75 %) from W8 to W24 (%)** |
|---|---|
| **Overall Response*** | 26.1 |
| | |
| **Flushes only** | 52.8 |
| **Pruritus only** | 28.6 |
| **Hamilton only** | 16.7 |
| **FIS only** | 8.0 |
| **Micturition frequency (pollakiuria) only** | 1.8 |
| **Stool frequency (diarrhea) only** | 18.4 |
| | |
| **Flushes or Pruritus** ** | 38.8 |
| **Hamilton or FIS** ** | 10.3 |

| | |
|---|---|
| * Overall response rate defined as proportion of patients reporting a ≥ 75% improvement from baseline on at least one handicap among pruritus, flushes, fatigue or depression. ** 2-handicap response rate defined as proportion of patients reporting a ≥ 75% improvement from baseline on at least one handicap from [flushes or pruritus], or on at least one handicap from [Hamilton or FIS]. Analysis based on missing data considered as failure (observed cases after W12). | |

Consequently, those patients identified as having systemic mastocytosis with severe mast cell mediator release associated handicap of pruritus and/or flushes represent a highly distinct subgroup from the overall population of patients with indolent forms of mastocytosis with handicap, for which masitinib can be expected to provide a clinically relevant therapeutic benefit. That is to say, systemic mastocytosis with severe mast cell mediator release associated handicap of pruritus and/or flushes is a highly predictive biomarker for masitinib treatment efficacy in severe systemic mastocytosis.

### Subpopulation of systemic mastocytosis with severe handicap in pruritus and flush frequency

In an alternative scenario, among those systemic mastocytosis patients from these pooled studies, 4 patients met the criteria for severe handicap at baseline, as defined as having handicaps of both pruritus and flushes (defined respectively as: pruritus score ≥9, and flushes frequency per week ≥8) with a total of 8 severe baseline handicaps reported. Assessment of treatment response on a handicap was defined as a ≥ 75% improvement from baseline on at least one handicap among pruritus or flushes. Results for overall response rates as well as relative changes in individual handicaps and response rates are presented in Table 3. Overall, at week 24 there was a response rate of 50.0%. An improving trend was observed for pruritus and flush variables, this subgroup size probably being too small to demonstrate statistical significance.

**Table 3. Masitinib treatment effect in pooled (AB06013 and AB04010 mastocytosis studies) cohort simulating a subpopulation of severe systemic mastocytosis (severe mast cell mediator release associated handicap at baseline of pruritus and flushes) - Overall response and individual handicap change.**

| | | Week 12 | Week 24 |
|---|---|---|---|
| Overall response ≥ 75%^{†} | | 1/8 (12.5%) | 4/8 (50.0%) |
| | | | |
| Number of flushes ≥ 8 | Relative change** | -50.3 (27.8) | -82.9 (27.9) |
| | p value | 0.125 | 0.125 |
| | | | |
| Pruritus score ≥ 9 | Relative change** | -12.9 (39.4) | -45.9 (63.9) |
| | p value | 0.750 | 0.375 |

| | | | |
|---|---|---|---|
| * Response rate missing data considered as failure. † Response defined as a ≥ 75% improvement from baseline on at least one handicap among pruritus or flushes. ** Mean relative change (%) with respect to baseline (SD), Last Observation Carried Forward. | | | |

### Example 3

Clinical data have shown that masitinib provides therapeutic benefit to a highly distinct subpopulation of mastocytosis patients, with the severity of mast cell mediator release associated handicaps serving as an independent predictor factor for treatment efficacy. These findings are without precedent because the predictive therapeutic significance of mast cell mediator release associated handicap severity in mastocytosis patients was previously unknown, in particular the predictive therapeutic significance of severe pruritus and severe flush handicaps.

A prospective, multicenter, randomized, placebo-controlled, parallel-group, phase 3 study, evaluated the efficacy and safety of masitinib (6 mg/kg/day administered orally in two daily intakes over 24-weeks with a double-blind extension phase possible) for the treatment of indolent systemic mastocytosis, smoldering systemic mastocytosis or cutaneous mastocytosis, in patients with mast cell mediator release symptoms that were refractory to conventional symptomatic treatment (see Example 1).

For analysis of masitinib's treatment-effect according to severity of mast cell mediator release associated handicaps a comparison was made between three patient groups derived from the overall study population:
- One group comprised patients diagnosed as having indolent systemic mastocytosis or smoldering systemic mastocytosis with severe mast cell mediator release symptoms. Severe mastocytosis was defined as patients having at least two of the six following mast cell mediator release associated handicaps at baseline: pruritus score ≥ 9, number of flushes per week ≥ 8, Hamilton rating scale for depression (HAMD-17) score ≥ 19, a fatigue score (asthenia) measured by Fatigue Impact Scale (FIS) ≥ 75, number of stools per day ≥ 4 (diarrhea), number of micturition per day ≥ 8 (pollakiuria), wherein at least one handicap is selected among pruritus, flushes, depression and fatigue (asthenia);
- A second group comprised patients diagnosed as having severe indolent systemic mastocytosis, severe smoldering systemic mastocytosis, or severe cutaneous mastocytosis. Hence, this subpopulation is less restricted with inclusion of cutaneous mastocytosis having severe mast cell mediator release symptoms at baseline. This group provides comparative analysis for patient selection according to variant of mastocytosis (i.e. systemic versus cutaneous);
- A third group comprised all randomized patients to study AB06006, corresponding therefore to the least restricted population, comprising patients with severe/non severe indolent and smoldering systemic mastocytosis, and severe/non severe cutaneous mastocytosis. This group provides comparative analysis based on patient selection according to severity of mast cell mediator release symptoms.

Results for various measures of treatment-effect, i.e. response rates and mean change relative to baseline, are presented in Tables 4 to 6 for the aforementioned patient groups. These data demonstrate that masitinib generates a clinical advantage in the subpopulation of severe systemic mastocytosis that is unexpectedly superior to other patient cohorts. The data presented in this example are in part taken from preliminary analysis (cut-off date November 2015) and as such represent a close approximation to the final, validated dataset.

Tables 4 and 5 show that masitinib generates a clinical advantage in the subpopulation of severe indolent/smoldering systemic mastocytosis that is unexpectedly superior to other patient cohorts. This is evidenced by (i) statistically significant masitinib treatment-effect when compared with placebo (according to the 4H[75%], 3H[75%], 2H[75%] response criteria) that is not apparent in the comparator patient populations, and (ii) the relative improvement in treatment-effect response between populations (according to the variable Factor Diff).

Table 4 provides a comparison between the subpopulations of severe indolent/smoldering systemic mastocytosis versus severe cutaneous/indolent/smoldering mastocytosis with response criteria according to severe baseline handicaps.

Table 5 provides a comparison between the subpopulations of severe indolent/smoldering systemic mastocytosis versus all randomized patients with response criteria according to moderate baseline handicaps. In this case the number of baseline handicaps considered is greater because of the less stringent thresholds on baseline handicap definition. This analysis is necessary for matched comparison with the overall study population, i.e. for inclusion of non-severe patients.

Table 6 provides a comparison between the subpopulations of indolent/smoldering systemic mastocytosis with severe baseline handicap versus all randomized patients with respect to mean relative change in pruritus score from baseline at week 24. The symptom of pruritus is well-recognized to be associated with mast cell activation in mastocytosis and therefore provides a direct measure of treatment-effect on mast cell activity. From this it is evident that the masitinib treatment-effect is substantially greater (approximately 6-fold) for the severe indolent/smoldering systemic mastocytosis cohort when compared with the overall population.

Taken together, these data demonstrate that masitinib generates a clinical advantage in the restricted population of severe systemic mastocytosis that is unexpectedly superior to other patient cohorts. These findings support the use of severity of mast cell mediator release associated handicaps as an independent predictor factor for masitinib treatment efficacy and patient selection from among non-aggressive forms of mastocytosis.

### Definitions:

† Comparison of severe baseline handicaps in given population, wherein said severe handicap is defined as: pruritus score ≥ 9, number of flushes per week ≥ 8, Hamilton rating scale for depression (HAMD-17) score ≥ 19, Fatigue Impact Scale (FIS) total score ≥ 75.
‡ Comparison of moderate baseline handicaps in given population, wherein said moderate handicap is defined as: pruritus score ≥ 6, number of flushes per week ≥7; HAMD-17 ≥ 10, FIS ≥40.
NS = non-significant.
Diff = absolute difference in response rates between treatment-arms of a given population (masitinib arm minus placebo arm).
Factor Diff = ratio of 'Diff' between compared populations showing relative superiority of response rate for severe systemic population over the comparator population.

Cumulative response was defined as the sum of actual responses between weeks 8 and 24 (5 time points with visits scheduled every 4 weeks) divided by the total number of possible responses over the same treatment period. Response was defined as a decrease in baseline symptom by 75%.
*4H[75%] (primary efficacy analysis outcome measure) = cumulative response on at least one of four baseline handicaps selected from pruritus, flushes, depression, and asthenia in the mITT population with missing data considered as failure. Response was defined as a decrease in baseline symptom by 75%.
**3H[75%] = cumulative response on pruritus or flushes or depression in the mITT population with missing data considered as failure. Response was defined as a decrease in baseline symptom by 75%.
***2H[75%] = cumulative response on pruritus or flushes in the mITT population with missing data considered as failure. Response was defined as a decrease in baseline symptom by 75%.
**** PF = patients with both pruritus and flushes handicap at baseline. Response was defined as a decrease in baseline symptom by 75%.

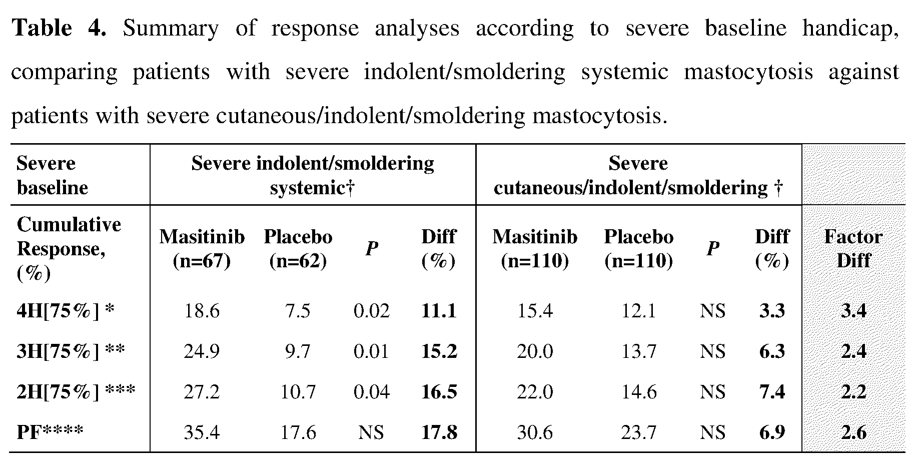

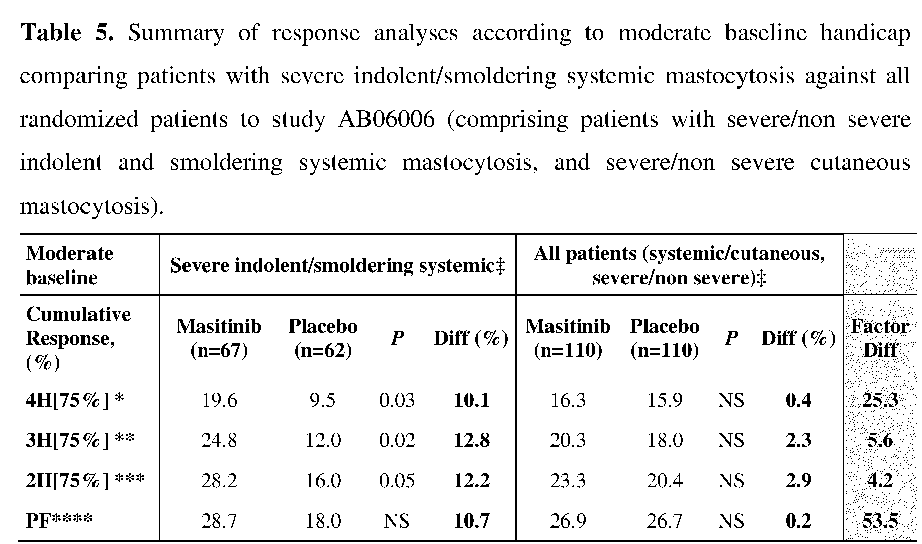

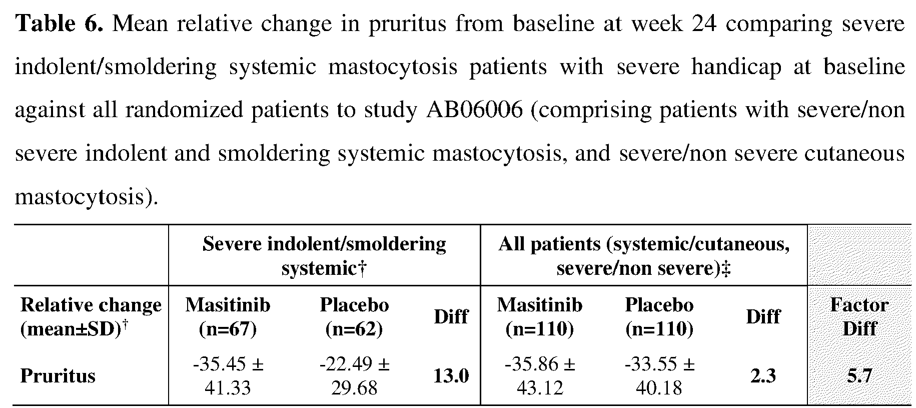

## Claims

1. Masitinib or a pharmaceutically acceptable salt or solvate thereof for use in the treatment of severe systemic mastocytosis in a human patient, wherein said severe systemic mastocytosis is associated with at least two mast cell mediator release associated handicaps selected from a pruritus score ≥ 9; a number of flushes per week ≥ 8; a Hamilton rating scale for depression (HAMD-17) score ≥ 19; a number of stools per day ≥ 4; a number of micturition per day ≥ 8; and a fatigue (asthenia) score measured by Fatigue Impact Scale (FIS) ≥ 75, and wherein at least two mast cell mediator release associated handicaps are selected from a pruritus score ≥ 9; a number of flushes per week ≥ 8; and a HAMD-17 score ≥ 19, wherein said systemic mastocytosis is smoldering systemic mastocytosis or indolent systemic mastocytosis.

2. Masitinib or a pharmaceutically acceptable salt or solvate thereof for use according to claim **1,** wherein said severe systemic mastocytosis is associated with a pruritus score ≥ 9 and a number of flushes per week ≥ 8.

3. Masitinib or a pharmaceutically acceptable salt or solvate thereof for use according to claim **1** or **2,** wherein said severe systemic mastocytosis is associated with the three following mast cell mediator release associated handicaps: a pruritus score ≥ 9; a number of flushes per week ≥ 8; and a HAMD-17 score ≥ 19.

4. Masitinib or a pharmaceutically acceptable salt or solvate thereof for use according to any one of claims **1** to **3,** wherein said severe systemic mastocytosis is further associated with the mast cell mediator release associated handicap of a fatigue (asthenia) score measured by FIS ≥ 75 or any equivalent cut-off determined using the Fatigue Severity Scale (FSS), Fatigue Symptom Inventory (FSI), Brief Fatigue Inventory (BFI) or Multidimensional Assessment of Fatigue (MAF).

5. Masitinib or a pharmaceutically acceptable salt or solvate thereof for use according to any one of claims **1** to **4,** wherein said smoldering systemic mastocytosis or indolent systemic mastocytosis is defined by the World Health Organization (WHO) diagnostic criteria.

6. Masitinib or a pharmaceutically acceptable salt or solvate thereof for use according to any one of claims **1** to **5,** wherein said smoldering systemic mastocytosis or indolent systemic mastocytosis relates to a disease for which any of the following four criteria are fulfilled at baseline or before baseline:
- presence of mast cells in any bone marrow biopsy or aspirate associated with at least one sign of abnormality, wherein said abnormal signs are selected from: more than 1% infiltration of mast cells in bone marrow; aggregates of more than 15 mast cells in bone marrow that can be described by the following words: nodules, seats, clusters, foci, or granuloma; more than 25% atypical mast cells in a sample of bone marrow; abnormal mast cells in the sample of bone marrow with microscopic testing that can be described by the following words: spindled, abnormal, atypical, fusiform, dystrophic, pathologic, dysmorphic; abnormal immunohistochemistry signs: mast cells in bone marrow express CD2 and/or CD25 present; and c-Kit point mutation at codon 816 (c-Kit 816) in bone marrow; or
- detection of c-Kit 816 in bone marrow without evidence of mast cells in bone marrow and detection of c-Kit 816 in skin biopsy justifying clonality; or
- presence of mast cells in bone marrow biopsy or aspirate without signs of mast cell abnormality; or
- excess of mast cells detected in digestive organs in addition to excess of mast cells in the skin.

7. Masitinib or a pharmaceutically acceptable salt or solvate thereof for use according to any one of claims **1** to **6,** wherein said human patient has a positive D816V c-Kit mutation status.

8. Masitinib or a pharmaceutically acceptable salt or solvate thereof for use according to any one of claims **1** to **7,** wherein said pharmaceutically acceptable salt or solvate of masitinib is masitinib mesilate.

9. Masitinib or a pharmaceutically acceptable salt or solvate thereof for use according to any one of claims **1** to **8,** wherein said masitinib or a pharmaceutically acceptable salt or solvate thereof, preferably masitinib mesilate, is for administration at a dose of 1.5, 3.0, 4.5, 6.0, 7.5, or 9.0 mg/kg/day (mg per kilo body weight per day); preferably at an initial dose of 3.0 mg/kg/day during at least 4 weeks, then 4.5 mg/kg/day during at least 4 weeks, and at 6 mg/kg/day thereafter, with each dose escalation being subjected to toxicity controls.

10. Masitinib or a pharmaceutically acceptable salt or solvate thereof for use according to any one of claims **1** to **9,** wherein said masitinib or a pharmaceutically acceptable salt or solvate thereof, preferably masitinib mesilate, is for administration in two daily intakes.

11. Masitinib or a pharmaceutically acceptable salt or solvate thereof for use according to any one of claims **1** to **10,** wherein said masitinib or a pharmaceutically acceptable salt or solvate thereof, preferably masitinib mesilate, is for oral administration.

12. Masitinib or a pharmaceutically acceptable salt or solvate thereof for use according to any one of claims **1** to **11,** wherein said masitinib or a pharmaceutically acceptable salt or solvate thereof, preferably masitinib mesilate, is comprised in a pharmaceutical composition in an amount of at least 50 mg and less than 600 mg, preferably of at least 100 mg and less than 400 mg.

## Patentansprüche

1. Masitinib oder ein pharmazeutisch annehmbares Salz oder Solvat davon zur Verwendung in der Behandlung schwerer systemischer Mastozytose bei einem menschlichen Patienten, wobei die schwere systemische Mastozytose mit mindestens zwei, mit der Freisetzung von Mastzellmediatoren assoziierten Beeinträchtigungen assoziiert ist, ausgewählt aus einem Pruritus-Score≥ 9; einer Anzahl von Flushes pro Woche ≥ 8; einem Score lt. Hamilton Rating Scale for Depression (HAMD-17) ≥ 19; einer Anzahl von Stuhlgängen pro Tag ≥ 4; einer Anzahl von Harnentleerungen pro Tag ≥ 8; und einem Fatigue- (Asthenie-) Score, lt. Fatigue Impact Scale (FIS) gemessen, ≥ 75, und wobei mindestens zwei mit der Freisetzung von Mastzellmediatoren assoziierte Beeinträchtigungen ausgewählt sind aus einem Pruritus-Score ≥ 9; einer Anzahl von Flushes pro Woche ≥ 8; und einem HAMD-17-Score ≥ 19, wobei die systemische Mastozytose schwelende systemische Mastozytose oder indolente systemische Mastozytose ist.

2. Masitinib oder ein pharmazeutisch annehmbares Salz oder Solvat davon zur Verwendung nach Anspruch **1,** wobei die schwere systemische Mastozytose mit einem Pruritus-Score ≥ 9 und einer Anzahl von Flushes pro Woche ≥ 8 assoziiert ist.

3. Masitinib oder ein pharmazeutisch annehmbares Salz oder Solvat davon zur Verwendung nach Anspruch **1** oder **2,** wobei die schwere systemische Mastozytose mit den drei folgenden, mit der Freisetzung von Mastzellmediatoren assoziierten Beeinträchtigungen assoziiert ist: einem Pruritus-Score ≥ 9; einer Anzahl von Flushes pro Woche ≥ 8; und einem HAMD-17-Score ≥ 19.

4. Masitinib oder ein pharmazeutisch annehmbares Salz oder Solvat davon zur Verwendung nach einem der Ansprüche **1** bis **3,** wobei die schwere systemische Mastozytose weiter mit der mit der Freisetzung von Mastzellmediatoren assoziierten Beeinträchtigung eines lt. FIS gemessenen Fatigue- (Asthenie-) Scores ≥ 75 oder einem äquivalenten Grenzwert assoziiert ist, der unter Verwendung der Fatigue Severity Scale (FSS), des Fatigue Symptom Inventory (FSI), des Brief Fatigue Inventory (BFI) oder der Multidimensional Assessment of Fatigue (MAF) gemessen wird.

5. Masitinib oder ein pharmazeutisch annehmbares Salz oder Solvat davon zur Verwendung nach einem der Ansprüche **1** bis **4,** wobei die schwelende systemische Mastozytose oder indolente systemische Mastozytose nach den Diagnosekriterien der Weltgesundheitsorganisation (WHO) definiert ist.

6. Masitinib oder ein pharmazeutisch annehmbares Salz oder Solvat davon zur Verwendung nach einem der Ansprüche **1** bis **5,** wobei sich die schwelende systemische Mastozytose oder indolente systemische Mastozytose auf eine Krankheit bezieht, bei der eines der folgenden vier Kriterien zu Ausgangswert oder vor Ausgangswert erfüllt ist:
- Vorhandensein von Mastzellen in einer Knochenmarksbiopsie oder einem Knochenmarkaspirat, die mit mindestens einem Zeichen einer Anomalie assoziiert ist, wobei die abnormalen Zeichen ausgewählt sind aus: mehr als 1 % Infiltration von Mastzellen im Knochenmark; Aggregate von mehr als 15 Mastzellen im Knochenmark, die mit den folgenden Worten beschrieben werden können: Knötchen, Sitze, Cluster, Foci oder Granulome; mehr als 25 % atypische Mastzellen in einer Knochenmarksprobe; abnormale Mastzellen in der Knochenmarksprobe bei mikroskopischer Untersuchung, die mit den folgenden Worten beschrieben werden können: spindelförmig, abnormal, atypisch, fusiform, dystrophisch, pathologisch, dysmorph; abnormale immunhistochemische Zeichen: Mastzellen im Knochenmark, die CD2 und/oder CD25 exprimieren, vorhanden; und c-Kit-Punktmutation an Codon 816 (c-Kit 816) im Knochenmark; oder
- Nachweis von c-Kit 816 im Knochenmark ohne Anzeichen von Mastzellen im Knochenmark, und Nachweis von c-Kit 816 in der Hautbiopsie, was für Klonalität spricht; oder
- Vorhandensein von Mastzellen in einer Knochenmarksbiopsie oder einem Knochenmarkaspirat ohne Zeichen einer Mastzellenanomalie; oder
- Überschuss an Mastzellen in den Verdauungsorganen nachgewiesen, zusätzlich zu einem Überschuss an Mastzellen in der Haut.

7. Masitinib oder ein pharmazeutisch annehmbares Salz oder Solvat davon zur Verwendung nach einem der Ansprüche **1** bis **6,** wobei der menschliche Patient einen positiven D816V c-Kit-Mutationsstatus aufweist.

8. Masitinib oder ein pharmazeutisch annehmbares Salz oder Solvat davon zur Verwendung nach einem der Ansprüche **1** bis **7,** wobei das pharmazeutisch annehmbare Salz oder Solvat von Masitinib Masitinib-Mesilat ist.

9. Masitinib oder ein pharmazeutisch annehmbares Salz oder Solvat davon zur Verwendung nach einem der Ansprüche **1** bis **8,** wobei das Masitinib oder ein pharmazeutisch annehmbares Salz oder Solvat davon, vorzugsweise Masitinib-Mesilat, zur Verabreichung in einer Dosis von 1,5, 3,0, 4,5, 6,0, 7,5 oder 9,0 mg/kg/Tag (mg pro Kilo Körpergewicht pro Tag) bestimmt ist; vorzugsweise in einer Anfangsdosis von 3,0 mg/kg/Tag während mindestens 4 Wochen, dann 4,5 mg/kg/Tag während mindestens 4 Wochen und danach 6 mg/kg/Tag, wobei jede Dosiseskalation Toxizitätskontrollen unterzogen wird.

10. Masitinib oder ein pharmazeutisch annehmbares Salz oder Solvat davon zur Verwendung nach einem der Ansprüche **1** bis **9,** wobei das Masitinib oder ein pharmazeutisch annehmbares Salz oder Solvat davon, vorzugsweise Masitinib-Mesilat, zur Verabreichung in zwei täglichen Einnahmen bestimmt ist.

11. Masitinib oder ein pharmazeutisch annehmbares Salz oder Solvat davon zur Verwendung nach einem der Ansprüche **1** bis **10,** wobei das Masitinib oder ein pharmazeutisch annehmbares Salz oder Solvat davon, vorzugsweise Masitinib-Mesilat, zur oralen Verabreichung bestimmt ist.

12. Masitinib oder ein pharmazeutisch annehmbares Salz oder Solvat davon zur Verwendung nach einem der Ansprüche **1** bis **11,** wobei das Masitinib oder ein pharmazeutisch annehmbares Salz oder Solvat davon, vorzugsweise Masitinib-Mesilat, in einer pharmazeutischen Zusammensetzung in einer Menge von mindestens 50 mg und weniger als 600 mg, vorzugsweise von mindestens 100 mg und weniger als 400 mg, umfasst ist.

## Revendications

1. Masitinib ou un sel ou solvate pharmaceutiquement acceptable de celui-ci pour son utilisation dans le traitement de la mastocytose systémique sévère chez un patient humain, dans laquelle ladite mastocytose systémique sévère est associée à au moins deux handicaps liés à la libération de médiateurs de mastocytes sélectionnés parmi un score de prurit ≥ 9 ; un nombre de bouffées vasomotrices par semaine ≥ 8 ; un score sur l'échelle d'évaluation de Hamilton pour la dépression (HAMD-17) ≥ 19 ; un nombre de selles par jour ≥ 4 ; un nombre de mictions par jour ≥ 8 ; et un score de fatigue (asthénie) mesuré par l'échelle FIS (*Fatigue Impact Scale*) ≥ 75, et dans laquelle au moins deux handicaps associés à la libération de médiateurs de mastocytes sont sélectionnés parmi un score de prurit ≥ 9 ; un nombre de bouffées vasomotrices par semaine ≥ 8 ; et un score HAMD-17 ≥ 19, dans laquelle ladite mastocytose systémique est une mastocytose systémique type *smoldering* ou une mastocytose systémique indolente.

2. Masitinib ou un sel ou solvate pharmaceutiquement acceptable de celui-ci pour son utilisation selon la revendication **1,** dans laquelle ladite mastocytose systémique sévère est associée à un score de prurit ≥ 9 et à un nombre de bouffées vasomotrices par semaine ≥ 8.

3. Masitinib ou un sel ou solvate pharmaceutiquement acceptable de celui-ci pour son utilisation selon la revendication **1** ou **2,** dans laquelle ladite mastocytose systémique sévère est associée aux trois handicaps liés à la libération des médiateurs de mastocytes suivants: un score de prurit ≥ 9 ; un nombre de bouffées vasomotrices par semaine ≥ 8 ; et un score HAMD-17 ≥ 19.

4. Masitinib ou un sel ou solvate pharmaceutiquement acceptable de celui-ci pour son utilisation selon l'une quelconque des revendications **1** à **3,** dans laquelle ladite mastocytose systémique sévère est en outre associée au handicap lié à la libération de médiateurs de mastocytes d'un score de fatigue (asthénie) mesuré par FIS ≥ 75 ou tout seuil équivalent déterminé en utilisant l'échelle FSS (*Fatigue Severity Scale*), l'inventaire FSI (*Fatigue Symptom Inventory*), l'inventaire BFI (*Brief Fatigue Inventory*) ou l'évaluation MAF (*Multidimensional Assessment of Fatigue).*

5. Masitinib ou un sel ou solvate pharmaceutiquement acceptable de celui-ci pour son utilisation selon l'une quelconque des revendications **1** à **4,** dans laquelle ladite mastocytose systémique type *smoldering* ou mastocytose systémique indolente est définie par les critères de diagnostic de l'Organisation mondiale de la santé (OMS).

6. Masitinib ou un sel ou solvate pharmaceutiquement acceptable de celui-ci pour son utilisation selon l'une quelconque des revendications **1** à **5,** dans laquelle ladite mastocytose systémique type *smoldering* ou mastocytose systémique indolente se rapporte à une maladie pour laquelle l'un quelconque des quatre critères suivants est rempli à la ligne de base ou avant la ligne de base :
- présence de mastocytes dans toute biopsie ou aspiration de moelle osseuse associée à au moins un signe d'anormalité, dans laquelle lesdits signes anormaux sont sélectionnés parmi : infiltration de plus de 1% de mastocytes dans la moelle osseuse ; agrégats de plus de 15 mastocytes dans la moelle osseuse qui peuvent être décrits par les mots suivants : nodules, sièges, grappes, foyers ou granulomes ; plus de 25 % de mastocytes atypiques dans un échantillon de moelle osseuse ; des mastocytes anormaux dans l'échantillon de moelle osseuse avec un test microscopique qui peut être décrit par les mots suivants : en forme de fuseau, anormal, atypique, fusiforme, dystrophique, pathologique, dysmorphique ; signes immunohistochimiques anormaux : les mastocytes dans la moelle osseuse expriment CD2 et/ou CD25 est présent ; et mutation ponctuelle de c-Kit au codon 816 (c-Kit 816) dans la moelle osseuse ; ou
- détection de c-Kit 816 dans la moelle osseuse sans preuve de mastocytes dans la moelle osseuse et détection de c-Kit 816 dans une biopsie de peau justifiant la clonalité ; ou
- présence de mastocytes dans une biopsie ou une aspiration de moelle osseuse sans signe d'anormalité des mastocytes ; ou
- excès de mastocytes détectés dans les organes digestifs en plus d'un excès de mastocytes dans la peau.

7. Masitinib ou un sel ou solvate pharmaceutiquement acceptable de celui-ci pour son utilisation selon l'une quelconque des revendications **1** à **6,** dans laquelle ledit patient humain a un statut de mutation D816V c Kit positif.

8. Masitinib ou un sel ou solvate pharmaceutiquement acceptable de celui-ci pour son utilisation selon l'une quelconque des revendications **1** à **7,** dans laquelle ledit sel ou solvate pharmaceutiquement acceptable de masitinib est le mésilate de masitinib.

9. Masitinib ou un sel ou solvate pharmaceutiquement acceptable de celui-ci pour son utilisation selon l'une quelconque des revendications **1** à **8,** dans laquelle ledit masitinib ou un sel ou solvate pharmaceutiquement acceptable de celui-ci, de préférence le mésilate de masitinib, est pour administration à une dose de 1,5, 3,0, 4,5, 6,0, 7,5 ou 9,0 mg/kg/jour (mg par kilo de poids corporel par jour) ; de préférence à une dose initiale de 3,0 mg/kg/jour pendant au moins 4 semaines, puis de 4,5 mg/kg/jour pendant au moins 4 semaines, et de 6 mg/kg/jour par la suite, chaque escalade de dose étant soumise à des contrôles de toxicité.

10. Masitinib ou un sel ou solvate pharmaceutiquement acceptable de celui-ci pour son utilisation selon l'une quelconque des revendications **1** à **9,** dans laquelle ledit masitinib ou un sel ou solvate pharmaceutiquement acceptable de celui-ci, de préférence le mésilate de masitinib, est pour administration en deux prises quotidiennes.

11. Masitinib ou un sel ou solvate pharmaceutiquement acceptable de celui-ci pour son utilisation selon l'une quelconque des revendications **1** à **10,** dans laquelle ledit masitinib ou un sel ou solvate pharmaceutiquement acceptable de celui-ci, de préférence le mésilate de masitinib, est pour administration orale.

12. Masitinib ou un sel ou solvate pharmaceutiquement acceptable de celui-ci pour son utilisation selon l'une quelconque des revendications **1** à **11,** dans laquelle ledit masitinib ou un sel ou solvate pharmaceutiquement acceptable de celui-ci, de préférence le mésilate de masitinib, est compris dans une composition pharmaceutique en une quantité d'au moins 50 mg et inférieure à 600 mg, de préférence d'au moins 100 mg et inférieure à 400 mg.
